(19) Europäisches Patentamt
European Patent Office
Office européen des brevets

(11) Veröffentlichungsnummer: **0 239 533 B1**

(12) # EUROPÄISCHE PATENTSCHRIFT

(45) Veröffentlichungstag der Patentschrift: **16.09.92**

(51) Int. Cl.5: **C07D 213/74**, C07D 213/75, A61K 31/44

(21) Anmeldenummer: **87810136.9**

(22) Anmeldetag: **11.03.87**

(54) **Pyridin-Derivate.**

(30) Priorität: **17.03.86 CH 1084/86**
**04.06.86 CH 2269/86**

(43) Veröffentlichungstag der Anmeldung:
**30.09.87 Patentblatt 87/40**

(45) Bekanntmachung des Hinweises auf die Patenterteilung:
**16.09.92 Patentblatt 92/38**

(84) Benannte Vertragsstaaten:
**AT BE CH DE ES FR GB GR IT LI LU NL SE**

(56) Entgegenhaltungen:
**DE-A- 2 717 280**
**US-A- 3 182 053**

(73) Patentinhaber: **CIBA-GEIGY AG**
**Klybeckstrasse 141**
**CH-4002 Basel(CH)**

(72) Erfinder: **von Sprecher, Georg, Dr.**
**Bettenstrasse 64**
**CH-4123 Allschwil(CH)**
Erfinder: **Waldmeier, Peter, Dr.**
**Kirchbündtenstrasse 26**
**CH-4107 Ettingen(CH)**

**EP 0 239 533 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Pyridin-Derivate der Formel

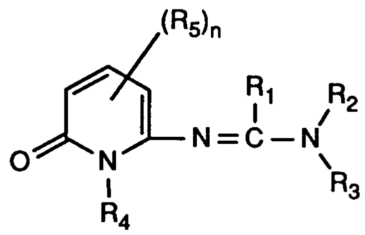

(I)

und ihre Tautomeren und/oder ihre Salze, worin $R_1$ Wasserstoff oder $C_1$-$C_7$-Alkyl bedeutet, einer der Reste $R_2$ und $R_3$ Wasserstoff, $C_1$-$C_7$-Alkyl, Phenyl- oder Naphthyl-$C_1$-$C_7$-alkyl, $C_3$-$C_6$-Alkenyl oder $C_3$-$C_6$-Alkinyl und der andere $C_1$-$C_7$-Alkyl, Phenyl- oder Naphthyl-$C_1$-$C_7$-alkyl, $C_3$-$C_6$-Alkenyl oder $C_3$-$C_6$-Alkinyl bedeutet, $R_4$ Wasserstoff, $C_1$-$C_7$-Alkyl oder Phenyl- oder Naphthyl-$C_1$-$C_7$-alkyl bedeutet, $R_5$ $C_1$-$C_7$-Alkyl oder Trifluormethyl bedeutet und der Index n 0, 1 oder 2 bedeutet, die Herstellung und Verwendung dieser Verbindungen und Salze, pharmazeutische Präparate, enthaltend eine Verbindung der Formel I oder ein Tautomeres und/oder ein Salz davon, und die Herstellung dieser pharmazeutischen Präparate.

Naphthyl ist 1- oder 2-Naphthyl. Bevorzugtes Phenyl-$C_1$-$C_7$-alkyl ist Phenyl-$C_1$-$C_4$-alkyl, insbesondere Benzyl und 2-Phenylethyl.

Die Verbindungen der Formel I und ihre Salze können in einem dynamischen Gleichgewicht mit entsprechenden tautomeren Formen stehen. Die 2-Oxo-dihydro-pyridine der Formel I können z.B., falls $R_4$ Wasserstoff bedeutet bzw. falls $R_3$ und $R_4$ jeweils Wasserstoff bedeuten, als 2-Hydroxy-pyridine der Formel

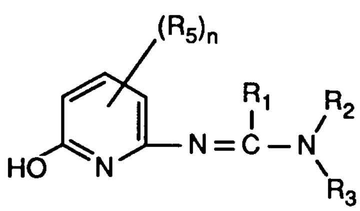

(Ia)

bzw.

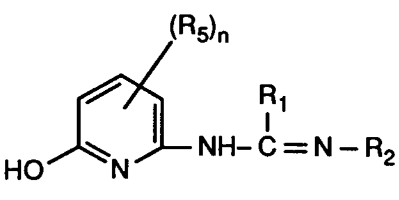

(Ib)

vorliegen. Ebenso können die Verbindungen der Formel I, falls $R_3$ Wasserstoff ist, im Gleichgewicht mit Tautomeren der Formel

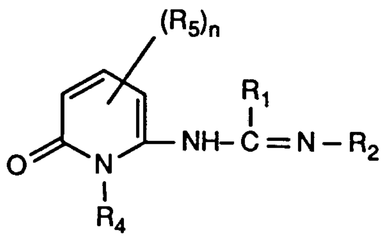

(Ic)

stehen.

Vor- und nachstehend umfasst der Ausdruck "Tautomere von Verbindungen der Formel I" entsprechende Verbindungen der Formeln Ia, Ib sowie Ic.

Bedeutet der Index n 2, kann $R_5$ gleiche oder ferner verschiedene Bedeutungen haben.

Verbindungen der Formel I können als, insbesondere pharmazeutisch verwendbare, Säureadditionssalze vorliegen. Diese werden beispielsweise mit starken anorganischen Säuren, wie Mineralsäuren, z.B. Schwefelsäure, Phosphorsäure oder Halogenwasserstoffsäuren, mit starken organischen Carbonsäuren, wie $C_1$-$C_4$-Alkancarbonsäuren, z.B. Essigsäure, wie gegebenenfalls ungesättigten Dicarbonsäuren, z.B. Oxal-, Malon-, Malein- oder Fumarsäure, oder wie Hydroxycarbonsäuren, z.B. Weinsäure oder Citronensäure, oder mit Sulfonsäuren, wie $C_1$-$C_4$-Alkan- oder gegebenenfalls substituierten Benzolsulfonsäuren, z.B. Methan- oder p-Toluolsulfonsäure, gebildet.

Entsprechende Säureadditionssalze können mit einem oder beiden basischen Zentren gebildet werden, wobei dementsprechend z.B. Pyridinium- und/oder bevorzugt Amidiniumsalze vorliegen.

Umfasst sind ferner für pharmazeutische Verwendungen nicht geeignete Salze, da diese beispielsweise für die Isolierung bzw. Reinigung freier erfindungsgemässer Verbindungen oder deren pharmazeutisch verwendbarer Salze verwendet werden können.

Die vor- und nachstehend verwendeten Allgemeindefinitionen haben, sofern nicht abweichend definiert, in erster Linie die folgenden Bedeutungen.

$C_1$-$C_7$-Alkyl ist z.B. Methyl, Ethyl, Propyl, Isopropyl, n-Butyl, Isobutyl, sek.-Butyl, tert.-Butyl und umfasst ferner entsprechende Pentyl-, Hexyl- und Heptylreste. Bevorzugt ist $C_1$-$C_4$-Alkyl.

In $C_3$-$C_6$-Alkenyl bzw. -Alkinyl ist die Mehrfachbindung in höherer als in der a-Stellung lokalisiert. Bevorzugt ist $C_3$-$C_4$-Alkenyl bzw. -Alkinyl, wie Allyl und Methallyl bzw. Propargyl.

Die Verbindungen der Formel I bzw. entsprechende tautomere Formen davon und ihre pharmazeutisch verwendbaren Säureadditionssalze weisen wertvolle pharmakologische Eigenschaften auf. So konnte für die erfindungsgemässe Verbindungsklasse ein neuartiges Wirkungsprofil ermittelt werden. Die Verbindungen der Formel I bzw. entsprechende tautomere Formen davon und ihre pharmazeutisch verwendbaren Salze erweisen sich als Catecholamin-O-methyl-transferase-(COMT-)Hemmer. Diese Eigenschaft kann in drei in vivo-Testsystemen nachgewiesen werden: durch die zu beobachtende Senkung der Homovanillinsäure im C. striatum der Ratte bzw. durch Hemmung der 3-Methoxy-tyramin-Akkumulation nach Monoaminoxidase-Hemmung im C. striatum der Ratte jeweils ab einer Dosis von 0,1 mg/kg nach i.p. Applikation, ferner im Rahmen einer Einzelzellableitung in der narkotisierten Ratte durch eine Erhöhung des Firing von locuscoeruleus-Zellen ab etwa 3 mg/kg.

Versuchsbeschreibungen

Bestimmung der Homovanillinsäure (HVA) im C. striatum der Ratte

Striata werden aus Rattenhirn präpariert und bis zur Analyse bei -20°C tiefgekühlt gelagert. Die Striata werden paarweise in 2 ml der mobilen Phase homogenisiert, die für die nachfolgend beschriebene HPLC-Trennung benötigt wird. Diese mobile Phase enthält pro Extrakt als internen Standard 1000 ng Vanillinsäure. Zellfragmente werden durch Zentrifugation entfernt. 50 bis 200 $\mu$l des Ueberstandes werden in ein BAS Flüssigchromatographiesystem (Bioanalytical Systems, W. Lafayette, Ind., USA) injiziert, welches mit einer $C_{18}$ $\mu$Bondapak Phasenumkehrsäule (Waters Ass., Milford, USA), mit einer TL3 elektrochemischen Detektorzelle und einem LC4 Kontrollsystem ausgestattet ist. Die Detektorzelle enthält $cp_w$ Graphitpaste, und das Potential wird auf +0,85 V gegenüber einer Ag/AgCl-Referenzelektrode eingestellt. Die mobile Phase, die 0,1 Mol/l Zitronensäure, 0,075 Mol/l Dinatriumhydrogenphosphat, 2,5 % Tetrahydrofuran und 0,05 Mol/l Natriumoctylsulfat enthält, wird mit Salzsäure auf pH=3 eingestellt. Die Säulentemperatur wird auf 28 bis 40°C und der Durchfluss auf 1 bis 1,3 ml/Min. gebracht, um eine optimale Trennung zu erreichen. Fünf

Tiere werden pro Gruppe eingesetzt.

Bestimmung der 3-Methoxy-tyramin(3-MT)-Anreicherung nach MAO-Hemmung durch Clorgyline

Ratten, denen die Testsubstanz 5 Minuten vor der Injektion von 10 mg/kg Clorgyline (s.c.) peroral bzw. intraperitoneal verabreicht wurde, werden 30 Minuten später durch Bestrahlen mit Mikrowellen (10 kW Stromleistung, 2450 MHz, Dauer 1,7 bis 1,8 Sekunden, Pueschner Mikrowellen-Energietechnik, Schwanewede/Bremen, Bundesrepublik Deutschland) getötet. Nach dem Abkühlen der Tiere, werden die Striata herauspräpariert und in einem Gemisch aus 2 ml 0,1 Mol/l Zitronensäure, 0,075 Mol/l Dinatriumhydrogenphosphat, 2,5 % Tetrahydrofuran und 0,05 mMol/l Natriumoctylsulfat, das mit Salzsäure auf pH = 3 eingestellt wird und dem als interner Standard 1000 ng Vanillinsäure zugesetzt wird, homogenisiert. Zellfragmente werden durch Zentrifugation abgetrennt. 50 bis 200 µl des Ueberstandes werden in ein BAS Flüssigchromatographiesystem (Bioanalytical Systems, W. Lafayette, Ind., USA) injiziert, welches ausgerüstet ist mit einer $C_{18}$ µBondapak Phasenumkehrsäule (Waters Ass., Milford, USA) und einem 5100A Coulometer-Detektor, Model ESA, mit einer Detektorzelle, Model 5010 (ESA Inc., Bedford, Mass., USA); Potential des Detektors 2: +0,45 V, Detektor 1 ausgeschaltet). Die mobile Phase besteht aus einem Citrat-Phosphat-Puffer(hergestellt durch Mischung von 0,1 M Zitronensäure und 0,1 M Dinatriumhydrogenphosphat bei pH = 3), dem 10 % Ethanol und 1,55 mM/l Natriumoctylsulfat zugesetzt sind; die Pumpgeschwindigkeit beträgt 1,3 ml/min.

Infolge der COMT-Inhibition wird der metabolische Abbau der in den Neuronen gebildeten und in der Folge von Nervenreizen freigesetzten Catecholamine, z.B. Dopamin, gehemmt, und es erfolgt eine Konzentrationserhöhung dieser Amine im synaptischen Spalt. Damit wird beispielsweise die Ursache von depressiven Erscheinungen und der Parkinson-Krankheit, z.B. der Dopamin-Mangel, weitgehend beseitigt. Bei Verwendung der erfindungsgemässen Verbindungen erfolgt gleichzeitig mit der COMT-Hemmung in den Neuronen eine Anreicherung des für die Methylierung benötigten S-Adenosylmethionins. Nach herkömmlicher Auffassung bedingt eine Erhöhung der S-Adenosylmethionin-Konzentration eine Steigerung der Lernfähigkeit.

Dementsprechend können die Verbindungen der Formel I bzw. Tautomere davon und deren pharmazeutisch vewendbare Salze z.B. als Pharmazeutika, wie als Nootropika, Antidepressiva und Antiparkinsonmittel, verwendet werden. Ein weiterer Gegenstand der Erfindung ist die Verwendung der erfindungsgemässen Verbindungen zur Herstellung von Arzneimitteln, insbesondere von Nootropika, Antidepressiva und Antiparkinsonmitteln, und zur therapeutischen und prophylaktischen Behandlung. Dabei kann auch die gewerbsmässige Herrichtung der Wirksubstanzen eingeschlossen sein.

Aus der DE-A-2,717,280 sind im Stand der Technik substituierte 1-Pyridyl-1,3,5-triaza-penta-1,4-diene mit akarizider Aktivität bzw. Aktivität gegenüber Milben bekannt. US-A-3 182 053 offenbart generisch gegebenenfalls am Pyridyl durch weitere Substituenten wie Alkyl und/oder OH-substituierte Pyridinformamidine; diese zeigen Wirksamkeit gegen Bakterien, Protozoen, Viren und Helminthen.

Die Erfindung betrifft insbesondere Verbindungen der Formel I und ihre Tautomeren und/oder ihre Salze, worin $R_1$ Wasserstoff oder $C_1$-$C_7$-Alkyl bedeutet, einer der Reste $R_2$ und $R_3$ Wasserstoff, $C_1$-$C_7$-Alkyl oder Phenyl- oder Naphthyl-$C_1$-$C_7$-alkyl und der andere $C_1$-$C_7$-Alkyl oder Phenyl- oder Naphthyl-$C_1$-$C_7$-alkyl bedeutet, $R_4$ Wasserstoff oder $C_1$-$C_7$-Alkyl bedeutet, $R_5$ $C_1$-$C_7$-Alkyl bedeutet und der Index n 0 oder 1 ist.

Die Erfindung betrifft insbesondere Verbindungen der Formel I und ihre Tautomeren und/oder ihre Salze, worin $R_1$ Wasserstoff oder $C_1$-$C_7$-Alkyl bedeutet, einer der Reste $R_2$ und $R_3$ Wasserstoff, $C_1$-$C_7$-Alkyl oder Phenyl- oder Naphthyl-$C_1$-$C_7$-alkyl und der andere $C_1$-$C_7$-Alkyl oder Phenyl- oder Naphthyl-$C_1$-$C_7$-alkyl bedeutet, $R_4$ Wasserstoff bedeutet und der Index n 0 ist.

Die Erfindung betrifft insbesondere Verbindungen der Formel I und ihre Tautomeren und/oder ihre Salze, worin einer der Reste $R_2$ und $R_3$ Wasserstoff, $C_1$-$C_7$-Alkyl oder Phenyl- oder Naphthyl-$C_1$-$C_7$-alkyl und der andere $C_1$-$C_7$-Alkyl oder Phenyl- oder Naphthyl-$C_1$-$C_7$-alkyl bedeutet.

Die Erfindung betrifft vor allem Verbindungen der Formel I und ihre Tautomeren und/oder ihre Salze, worin $R_1$ Wasserstoff oder $C_1$-$C_4$-Alkyl, wie Methyl, bedeutet, $R_2$ und $R_3$ unabhängig voneinander $C_1$-$C_4$-Alkyl, wie Propyl, bedeuten oder $R_2$ $C_1$-$C_4$-Alkyl, wie Methyl, bedeutet und $R_3$ Phenyl-$C_1$-$C_4$-alkyl, wie 2-Phenylethyl, bedeutet, $R_4$ Wasserstoff ist und der Index n 0 ist.

Die Erfindung betrifft vor allem Verbindungen der Formel I und ihre Tautomeren und/oder ihre Salze, worin $R_1$ Wasserstoff oder $C_1$-$C_4$-Alkyl, wie Methyl, bedeutet, $R_2$ und $R_3$ unabhängig voneinander $C_1$-$C_4$-Alkyl, wie Propyl, bedeuten oder $R_2$ $C_1$-$C_4$-Alkyl, wie Methyl, bedeutet und $R_3$ Phenyl-$C_1$-$C_4$-alkyl, wie 2-Phenylethyl, bedeutet, $R_4$ $C_1$-$C_4$-Alkyl, wie Methyl, ist und der Index n 0 ist.

Die Erfindung betrifft vor allem Verbindungen der Formel I und ihre Tautomeren und/oder ihre Salze,

worin $R_1$ und $R_4$ Wasserstoff bedeuten, $R_2$ und $R_3$ unabhängig voneinander $C_1$-$C_4$-Alkyl, wie Propyl, bedeuten und der Index n 0 ist.

Die Erfindung betrifft vor allem Verbindungen der Formel I und ihre Tautomeren und/oder ihre Salze, worin $R_1$ Wasserstoff bedeutet, $R_4$ $C_1$-$C_4$-Alkyl, wie Methyl, bedeutet, $R_2$ und $R_3$ unabhängig voneinander $C_1$-$C_4$-Alkyl, wie Propyl, bedeuten und der Index n 0 ist.

Die Erfindung betrifft vor allem Verbindungen der Formel I und ihre Tautomeren und/oder ihre Salze, worin $R_1$ Methyl ist, $R_4$ Wasserstoff bedeutet, $R_2$ und $R_3$ unabhängig voneinander $C_1$-$C_4$-Alkyl, wie Propyl, bedeuten und der Index n 0 ist.

Die Erfindung betrifft insbesondere die in den Beispielen genannten neuen Verbindungen der Formel I und Verfahren zu ihrer Herstellung.

Ebenfalls Gegenstand der Erfindung sind Verfahren zur Herstellung der erfindungsgemässen Verbindungen. Die Herstellung von Verbindungen der Formel I oder ihrer Tautomeren und/oder ihrer Salze erfolgt in an sich bekannter Weise und ist z.B. dadurch gekennzeichnet, dass man

a) eine Verbindung der Formel

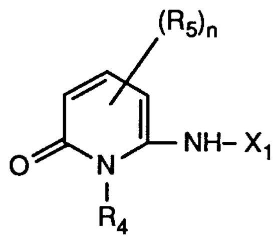

(IIa)

oder ein Tautomeres davon mit einer Verbindung der Formel

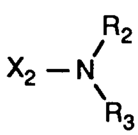

(IIb),

worin einer der Reste $X_1$ und $X_2$ für eine Gruppe der Formel-CO-$R_1$ steht und der andere Wasserstoff oder eine Gruppe der Formel-CO-$Z_1$ ist, worin $Z_1$ einen abspaltbaren Rest bedeutet, oder einem Tautomeren, Salz und/oder Acetal davon umsetzt oder

b) in einer Verbindung der Formel

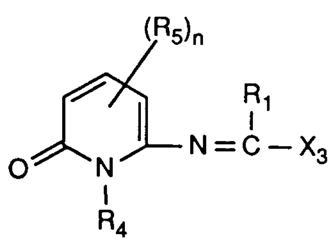

(III)

oder einem Tautomeren und/oder Salz davon, worin $X_3$ einen in

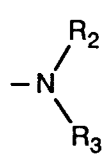

überführbaren Rest bedeutet, $X_3$ in

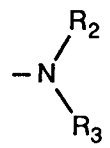

überführt oder

c) eine Verbindung der Formel

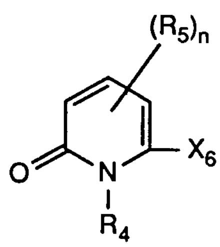

(Va)

oder ein Tautomeres oder Salz davon mit einer Verbindung der Formel

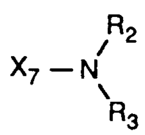

(Vb)

oder einem Tautomeren oder einem Salz davon, wobei $X_6$ für die Gruppe $-N=CR_1-NH-X_6$ steht und $X_7$ für Wasserstoff steht oder $X_6$ für $-NH_2$ steht und $X_7$ für die Gruppe $-CR_1=N-X_6$ steht und $X_6$ für eine Abgangsgruppe steht, umsetzt oder

d) zur Herstellung einer Verbindung der Formel I oder eines Tautomeren und/oder Salzes davon, worin $R_4$ Wasserstoff bedeutet, in einer Verbindung der Formel

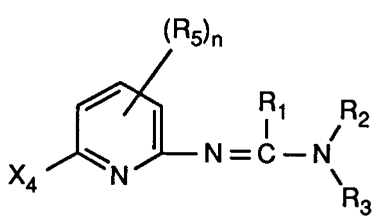

(IV)

oder einem Salz davon, worin $X_4$ für geschütztes Hydroxy steht, die Hydroxyschutzgruppe abspaltet und gewünschtenfalls jeweils eine verfahrensgemäss oder auf andere Weise erhältliche Verbindung der Formel I oder ein Tautomeres davon in eine andere Verbindung der Formel I bzw. ein Tautomeres davon überführt, ein verfahrensgemäss erhältliches Isomerengemisch in die Komponenten auftrennt und/oder eine verfahrensgemäss erhältliche freie Verbindung der Formel I bzw. ein Tautomeres davon in ein Salz überführt oder ein verfahrensgemäss erhältliches Salz in die freie Verbindung der Formel I bzw. ein Tautomeres davon oder in ein anderes Salz überführt.

Die vor- und nachstehend in den Varianten beschriebenen Umsetzungen werden in an sich bekannter Weise durchgeführt, z.B. in Ab- oder üblicher Weise in Anwesenheit eines geeigneten Lösungs- oder Verdünnungsmittels oder eines Gemisches derselben, wobei man je nach Bedarf unter Kühlen, bei Raumtemperatur oder unter Erwärmen, z.B. in einem Temperaturbereich von -80° bis zur Siedetemperatur

des Reaktionsmediums, vorzugsweise von -10° bis +100°C, und, falls erforderlich, in einem geschlossenen Gefäss, unter Druck, in einer Inertgasatmosphäre und/oder unter wasserfreien Bedingungen arbeitet.

Das vor- und nachstehend aufgeführte Ausgangsmaterial der Formeln IIa, IIb, III, IV, Va und Vb, das für die Herstellung der Verbindungen der Formel I oder ihrer Tautomeren und/oder ihrer Salze entwickelt wurde, ist bekannt oder kann nach an sich bekannten Methoden, z.B. analog den vorstehend beschriebenen Verfahrensvarianten, hergestellt werden.

Salze der Ausgangsmaterialien der Formeln IIa, IIb, III, IV, Va und Vb sind in erster Linie entsprechende Säureadditionssalze, da diese Ausgangsverbindungen mindestens ein basisches Zentrum aufweisen.

Geeignete Säuren für die Salzbildung sind beispielsweise starke anorganische Säuren, wie Mineralsäuren, z.B. Schwefelsäure, Phosphorsäure oder Halogenwasserstoffsäuren, starke organische Carbonsäuren, wie $C_1$-$C_4$-Alkancarbonsäuren, z.B. Eisessig, wie gegebenenfalls ungesättigte Dicarbonsäuren, z.B. Oxal-, Malon-, Malein- oder Fumarsäure, oder wie Hydroxycarbonsäuren, z.B. Weinsäure oder Citronensäure, oder Sulfonsäuren, wie $C_1$-$C_4$-Alkan- oder gegebenenfalls substituierte Benzolsulfonsäuren, z.B. Methan- oder p-Toluolsulfonsäure.

Die 2-Oxo-dihydro-pyridin-Derivate der Formeln IIa, III und Va können ebenfalls in Form entsprechender tautomerer 2-Hydroxypyridine vorliegen.

Acetale von Verbindungen der Formeln IIa und IIb, worin einer der Reste $X_1$ und $X_2$ für die Gruppe der Formel -CO-$R_1$ und der andere für Wasserstoff steht, sind solche Verbindungen, in denen die Carbonylfunktion mit einem ein- oder zweiwertigen aliphatischen Alkohol, wie einem $C_1$-$C_7$-Alkanol oder $C_2$-$C_5$-Alkandiol, acetalisiert bzw. ketalisiert ist.

Variante a):

Ein abspaltbarer Rest $Z_1$ ist beispielsweise reaktionsfähiges verestertes Hydroxy oder gegebenenfalls verethertes Hydroxy bzw. Mercapto. Reaktionsfähiges verestertes Hydroxy ist insbesondere mit einer starken anorganischen Säure oder organischen Sulfonsäure verestertes Hydroxy, beispielsweise Halogen, wie Chlor, Brom oder Iod, Sulfonyloxy, wie Hydroxysulfonyloxy, Halogensulfonyloxy, z.B. Fluorsulfonyloxy, gegebenenfalls, z.B. durch Halogen, substituiertes $C_1$-$C_7$-Alkansulfonyloxy, z.B. Methan- oder Trifluormethansulfonyloxy, $C_5$-$C_7$-Cycloalkansulfonyloxy, z.B. Cyclohexansulfonyloxy, oder gegebenenfalls, z.B. durch $C_1$-$C_7$-Alkyl oder Halogen, substituiertes Benzolsulfonyloxy, z.B. p-Bromphenyl- oder p-Toluolsulfonyloxy. Verethertes Hydroxy ist z.B. gegebenenfalls, z.B. durch Phenyl, substituiertes $C_1$-$C_7$-Alkoxy, wie Methoxy, Ethoxy oder Benzyloxy, während als verethertes Mercapto z.B. $C_1$-$C_7$-Alkylthio, wie Methyl- oder Ethylthio, in Frage kommt. $Z_1$ bedeutet bevorzugt Halogen, wie Chlor, oder $C_1$-$C_4$-Alkoxy, wie Methoxy oder Ethoxy.

Die Umsetzung von Verbindungen der Formeln IIa und IIb, worin einer der Reste $X_1$ und $X_2$ für eine Gruppe der Formel -CO-$R_1$ und der andere für Wasserstoff steht, wird insbesondere in Gegenwart eines Kondensationsmittels, wie eines Dehydratisierungsmittels oder eines Anhydrids einer anorganischen Säure, durchgeführt.

Als Dehydratisierungsmittel können insbesondere Carbodiimide, z.B. N,N'-Di-$C_1$-$C_4$-alkyl- oder N,N'-Di-$C_5$-$C_7$-cycloalkyl-carbodiimide, wie N,N'-Dicyclohexyl-carbodiimid, vorteilhaft unter Zusatz von N-Hydroxysuccinimid oder gegebenenfalls, z.B. durch Halogen, $C_1$-$C_7$-Alkyl oder $C_1$-$C_7$-Alkoxy, substituiertem N-Hydroxy-benzotriazol oder N-Hydroxy-5-norbornen-2,3-dicarboxamid, ferner geeignete Carbonylverbindungen, z.B. N,N'-Carbonyldiimidazol, geeignete 1,2-Oxazoliumverbindungen, z.B. 2-Ethyl-5-phenyl-1,2-oxazolium-3'-sulfonat oder 2-tert-Butyl-5-methyl-isoxazolium perchlorat, geeignete Acylaminoverbindungen, z.B. 2-Ethoxy-1-ethoxycarbonyl-1,2-dihydrochinolin, oder geeignete Phosphorylcyanamide bzw. -azide, z.B. Diethylphosphorylcyanamid oder Diphenylphosphorylazid, ferner Triphenylphosphan-disulfid oder 1-$C_1$-$C_4$-Alkyl-2-halogen-pyridinium-halogenide, z.B. 1-Methyl-2-chlor-pyridinium-iodid, eingesetzt werden.

Als Beispiele für Anhydride anorganischer Säuren seien Phosphorpentoxid, Phosphoroxyhalogenide, wie Phosphoroxychlorid, Phosgen und Thionylchlorid genannt.

Vorzugsweise werden solche Verbindungen der Formeln IIa und IIb eingesetzt, worin einer der Reste $X_1$ und $X_2$ für eine in acetalisierter bzw. ketalisierter Form vorliegende Gruppe der Formel-CO-$R_1$ steht und der andere Wasserstoff bedeutet.

Bei der Reaktion von Verbindungen der Formel IIa und IIb, worin einer der Reste $X_1$ und $X_2$ für eine Gruppe der Formel -CO-$R_1$ und der andere für eine Gruppe der Formel -CO-$Z_1$ steht und $Z_1$ einen abspaltbaren Rest bedeutet, werden bevorzugt solche Ausgangsmaterialien verwendet, worin $Z_1$ Halogen, wie Chlor, bedeutet. Die Umsetzung wird insbesondere unter Erwärmen, z.B. in einem Temperaturbereich von 50°C bis zur Siedetemperatur des Reaktionsmediums, durchgeführt.

Das Ausgangsmaterial der Formeln IIa und IIb ist bekannt oder kann in an sich bekannter Weise hergestellt werden, beispielsweise durch N-Acylierung von Aminen der Formeln

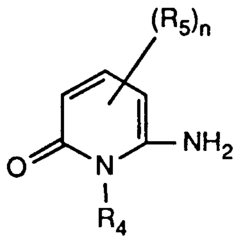 (IIIa) bzw. 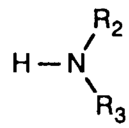 (IIc).

Variante b):

Ein in

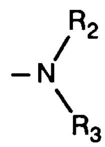

überführbarer Rest $X_3$ ist beispielsweise Amino.

Entsprechende Verbindungen der Formel III' (Verbindungen III, worin $X_3$ Amino ist) können beispielsweise mit einem $R_2$ und $R_3$ zugrundeliegendem Alkylierungsmittel, wie einem von $R_2$-OH und $R_3$-OH oder einem reaktionsfähigen veresterten Derivat davon oder einer entsprechenden Carbonylverbindung abgeleiteten Alkylierungsmittel, alkyliert werden. Derartige Alkylierungsreagentien sind beispielsweise entsprechende Halogenide, Sulfate oder Sulfonate, z.B. der Formel $R_2$-$Z_2$ und $R_3$-$Z_2$, wobei $Z_2$ z.B. Halogen oder Sulfonyloxy, wie $C_1$-$C_7$-Alkan- oder gegebenenfalls substituiertes Benzolsulfonyloxy, z.B. Methan- oder p-Toluolsulfonyloxy, bedeutet.

Die Alkylierung entsprechender Verbindungen der Formel III' wird insbesondere in Gegenwart einer Base durchgeführt.

Als Basen kommen beispielsweise Alkalimetallhydroxide, -hydride, -amide, -alkanolate, -carbonate, -triphenylmethylide, -di-$C_1$-$C_7$-alkylamide, -amino-$C_1$-$C_7$-alkylamide oder -$C_1$-$C_7$-alkylsilylamide, Naphthalinamine, $C_1$-$C_7$-Alkylamine, basische Heterocyclen, Ammoniumhydroxide sowie carbocyclische Amine in Frage. Beispielhaft seien Lithium-hydroxid, Natrium-hydroxid, -hydrid, -amid, -ethylat, Kalium-tert-butylat, -carbonat, Lithiumtriphenylmethylid, -diisopropylamid, Kalium-3-(aminopropyl)-amid, -bis-(trimethylsilyl)-amid, Dimethylaminonaphthalin, Di- oder Triethylamin, Pyridin, Benzyltrimethyl-ammoniumhydroxid, 1,5-Diaza-bicyclo[4.3.0]non-5-en (DBN) sowie 1,8-Diaza-bicyclo[5.4.0]undec-7-en (DBU) genannt.

Werden als Alkylierungsmittel z.B. Carbonylverbindungen eingesetzt,kann die Reaktion z.B. in Gegenwart eines Reduktionsmittels, z.B. mit Ameisensäure und Formamid analog der Leuckart-Wallach-Reaktion, durchgeführt werden.

$X_3$ in der Formel III kann ferner für eine Abgangsgruppe wie reaktionsfähiges verestertes Hydroxy oder gegebenenfalls verethertes Hydroxy bzw. Mercapto stehen. Reaktionsfähiges verestertes Hydroxy ist insbesondere mit einer starken anorganischen Säure oder organischen Sulfonsäure verestertes Hydroxy, beispielsweise Halogen, wie Chlor, Brom oder Iod, Sulfonyloxy, wie Hydroxysulfonyloxy, Halogensulfonyloxy, z.B. Fluorsulfonyloxy, gegebenenfalls, z.B. durch Halogen, substituiertes $C_1$-$C_7$-Alkansulfonyloxy, z.B. Methan- oder Trifluormethansulfonyloxy, $C_5$-$C_7$-Cycloalkansulfonyloxy, z.B. Cyclohexansulfonyloxy, oder gegebenenfalls, z.B. durch $C_1$-$C_7$-Alkyl oder Halogen, substituiertes Benzolsulfonyloxy, z.B. p-Bromphenyl- oder p-Toluolsulfonyloxy. Verethertes Hydroxy ist z.B. gegebenenfalls, z.B. durch Phenyl, substituiertes $C_1$-$C_7$-Alkoxy, wie Methoxy, Ethoxy oder Benzyloxy, während als verethertes Mercapto z.B. $C_1$-$C_7$-Alkylthio, wie Methyl- oder Ethylthio, in Frage kommt. $X_3$ bedeutet als Abgangsgruppe bevorzugt Halogen, wie Chlor, oder $C_1$-$C_4$-Alkoxy, wie Methoxy oder Ethoxy.

Entsprechende Verbindungen der Formel III, worin $X_3$ für eine Abgangsgruppe steht, werden mit einem Amin der Formel

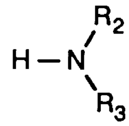

(IIc) oder einem Salz davon umgesetzt.

Das Ausgangsmaterial der Formel III, worin $X_3$ Amino bedeutet (Ausgangsmaterial der Formel III'), ist z.B. zugänglich durch Umsetzung einer Verbindung der Formel

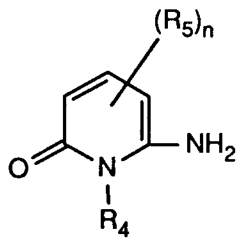

(III'a)

oder eines Tautomeren und/oder Salzes davon mit Ammoniak.

Zu Ausgangsverbindungen der Formel III, worin $X_3$ für eine Abgangsgruppe steht, kann man z.B. gelangen, indem man eine Verbindung der Formel III'a zunächst mit einem Halogenierungsreagenz, wie Phosgen, behandelt (und so zu Verbindungen III gelangt, worin $X_3$ = Halogen) und die entsprechenden resultierenden Verbindungen der Formel III $X_3$ = Halogen) z.B. mit einem gewünschten Alkohol bzw. Mercaptan umsetzt.

Variante d):

Als geschütztes Hydroxy $X_4$ kommt beispielsweise verestertes, verethertes oder acetalisiertes Hydroxy in Betracht, wie Acyloxy, Silyloxy, gegebenenfalls substituiertes Alkoxy oder Tetrahydropyranyloxy. Der Acylrest in Acyloxy bedeutet beispielsweise gegebenenfalls, z.B. durch Halogen, substituiertes $C_2$-$C_5$-Alkanoyl bzw. Benzoyl, wie Acetyl, Monochloracetyl oder Benzoyl, oder gegebenenfalls durch einen Phenylrest substituiertes $C_2$-$C_5$-Alkoxycarbonyl, wie Ethoxy-, tert.-Butyloxy-, Benzyloxy-, 2-Brombenzyloxy- oder 4-Methoxybenzyloxy-carbonyl. Silyl in Silyloxy ist z.B. Tri-$C_1$-$C_4$-alkyl-silyl, wie Trimethylsilyl oder tert.-Butyl-dimethylsilyl. Gegebenenfalls substituiertes Alkyl gemäss entsprechendem Alkoxy steht beispielsweise für gegebenenfalls, z.B. durch einen Phenylrest, substituiertes $C_1$-$C_4$-Alkyl, wie tert.-Butyl, Benzyl oder 3-Brombenzyl.

Die Abspaltung der jeweiligen Hydroxyschutzgruppe erfolgt in an sich bekannter Weise, beispielsweise durch Hydrolyse, Acidolyse, Reduktion, Hydrazinolyse oder Behandeln mit Thioharnstoff.

So werden beispielsweise $C_2$-$C_5$-Alkanoyl-, Benzoyl-, Ethoxycarbonyl-, Benzoyloxycarbonyl-, Tetrahydropyranyl- oder Silylreste durch Hydrolyse, insbesondere in Gegenwart einer Säure oder in erster Linie einer Base, abgespalten, während z.B. 2-Brombenzyloxycarbonyl-, Benzyloxycarbonyl-, Benzyl-, 3-Brombenzyl- oder tert.-Butylreste durch Acidolyse, z.B. durch Behandeln mit einer starken Säure, wie Salzsäure, Bromwasserstoffsäure/Eisessig, Flussäure oder Trifluoressigsäure, abgespalten werden. Aus Benzyloxy kann Hydroxy ebenfalls durch Reduktion, z.B. durch katalytische Hydrierung, vorteilhaft in Gegenwart eines Hydrierungskatalysators, oder durch Behandeln mit Natrium in flüssigem Ammoniak, freigesetzt werden.

Die Verbindungen der Formel IV sind beispielsweise erhältlich durch Umsetzung einer Verbindung der Formel

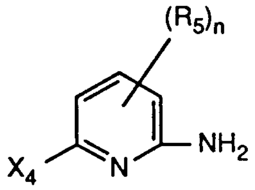

(IVa)

oder eines Salzes davon mit einer Verbindung der Formel

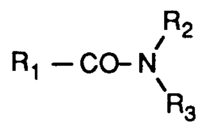

(IVb) oder einem Acetal bzw. Ketal davon, wobei analog der Verfahrensvariante a) vorgegangen wird.

Variante c):

Als Abgangsgruppe $X_5$ kommt vorzugsweise Cyano in Frage.

Das Ausgangsmaterial der Formel Vb, worin $X_7$ für die Gruppe $-CR_1=N-X_5$ steht und $X_5$ Cyano bedeutet, kann z.B. erhalten werden, indem man eine Verbindung der Formel

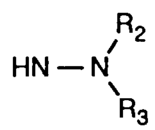

(IIc) oder einem Salz davon mit einer Verbindung der Formel

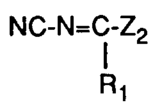

(Vc), worin $Z_2$ eine Abgangsgruppe, z.B. $C_1$-$C_4$-Alkoxy, bedeutet, umsetzt.

Zur Herstellung von Verbindungen der Formel Va, worin $X_5$ für $-N=CR_1-NH-X_5$ und $X_5$ Cyano bedeutet, geht man beispielsweise von einer Verbindung der Formel IIIa aus und setzt diese mit einer Verbindung der Formel $X_5-N=CR_1-NH_2$ (Vd), gegebenenfalls unter Erwärmen, um.

Die Erfindung betrifft ebenfalls die nach den vorstehenden Verfahrensvarianten erhältlichen neuen Verbindungen.

Eine erfindungsgemäss oder auf andere Weise erhältliche Verbindung der Formel I oder ein Tautomeres davon kann in an sich bekannter Weise in eine andere Verbindung der Formel I bzw. eine tautomere Form davon überführt werden.

Bedeutet einer der Reste $R_2$ und $R_3$ Wasserstoff, können entsprechende Verbindungen der Formel I und ihre Tautomeren und/oder Salze in an sich bekannter Weise N-alkyliert werden. Die N-Alkylierung erfolgt z.B. mit einem Alkylhalogenid, z.B. einem -bromid oder -iodid, Alkansulfonat, z.B. Methansulfonat, einem p-Toluolsulfonat oder einem Di-alkylsulfat, z.B. Dimethylsulfat, vorzugsweise unter basischen Bedingungen, wie in Gegenwart von Natronlauge oder Kalilauge, und vorteilhaft eines Phasentransfer-Katalysators, wie von Tetrabutylammoniumbromid oder Benzyltrimethylammoniumchlorid, wobei indes stärker basische Kondensationsmittel, wie Alkalimetallamide, -hydride oder -alkoholate, z.B. Natriumamid, Natriumhydrid oder Natriumethanolat, erforderlich sein können.

Ebenso können die Verbindungen der Formel I und ihre Tautomere und/oder Salze durch Behandeln mit einem entsprechenden Amin umamidiniert werden.

Verbindungen der Formel I und Tautomere und/oder Salze davon, worin $R_4$ Wasserstoff bedeutet, können mit Hilfe eines geeigneten Alkylierungsmittels Ring-N-alkyliert werden.

Enthalten die Verbindungen der Formel I $C_3$-$C_6$-Alkenyl- oder $C_3$-$C_6$-Alkinylgruppierungen, können diese in an sich bekannter Weise in entsprechende gesättigte Reste überführt werden. So erfolgt beispielsweise die Hydrierung von Mehrfachbindungen durch katalytische Hydrierung in Gegenwart von Hydrierungskatalysatoren, wobei hierfür z.B. Edelmetalle bzw. deren Derivate, z.B. Oxide, geeignet sind, wie Nickel, Raney-Nickel, Palladium oder Platinoxid, die gegebenenfalls auf Trägermaterialien, z.B. auf Kohle oder Calciumcarbonat, aufgezogen sein können. Die Hydrierung kann vorzugsweise bei Drucken zwischen 1 und etwa 100 at durchgeführt werden.

Salze von Verbindungen der Formel I oder deren Tautomeren können in an sich bekannter Weise hergestellt werden. So erhält man beispielsweise Säureadditionssalze von Verbindungen der Formel I oder Tautomeren davon durch Behandeln mit einer Säure oder einem geeigneten Ionenaustauscherreagenz. Salze können in üblicher Weise in die freien Verbindungen überführt werden, Säureadditionssalze z.B. durch Behandeln mit einem geeigneten basischen Mittel.

Je nach Verfahrensweise bzw. Reaktionsbedingungen können die erfindungsgemässen Verbindungen mit salzbildenden, insbesondere basischen Eigenschaften, in freier Form oder bevorzugt in Form von Salzen erhalten werden.

Infolge der engen Beziehung zwischen der neuen Verbindung I in freier Form und in Form ihrer Salze sind im Vorausgegangenen und nachfolgend unter der freien Verbindung I oder ihren Salzen sinn- und zweckgemäss gegebenenfalls auch die entsprechenden Salze bzw. die freie Verbindung I zu verstehen.

Die neuen Verbindungen I einschliesslich ihrer Salze von salzbildenden Verbindungen können auch in Form ihrer Hydrate erhalten werden oder andere zur Kristallisation verwendete Lösungsmittel einschliessen.

Die neuen Verbindungen I können, je nach der Wahl der Ausgangsstoffe und Arbeitsweisen, in Form eines der möglichen Isomeren oder als Gemisch derselben, z.B. je nach der Anzahl der asymmetrischen Kohlenstoffatome, als reine optische Isomere, wie Antipoden, oder als Isomerengemische, wie Racemate, Diastereoisomerengemische oder Racematgemische, vorliegen.

Erhaltene Racematgemische können auf Grund der physikalisch-chemischen Unterschiede der Bestandteile in bekannter Weise in die reinen Isomeren oder Racemate getrennt aufgetrennt werden, beispielsweise durch fraktionierte Kristallisation.

Erhaltene Racemate lassen sich ferner nach bekannten Methoden in die optischen Antipoden zerlegen, beispielsweise durch Umkristallisation aus einem optisch aktiven Lösungsmittel, Chromatographie an chiralen Adsorbentien, mit Hilfe von geeigneten Mikroorganismen, durch Spaltung mit spezifischen, immobilisierten Enzymen, über die Bildung von Einschlussverbindungen, z.B. unter Verwendung chiraler Kronenether, wobei nur ein Enantiomeres komplexiert wird, oder durch Ueberführung in diastereomere Salze, z.B. durch Umsetzung eines basischen Endstoffracemats mit einer optisch aktiven Säure, wie Carbonsäure, z.B. Wein- oder Aepfelsäure, oder Sulfonsäure, z.B. Camphersulfonsäure, und Trennung des auf diese Weise erhaltenen Diastereomerengemisches, z.B. auf Grund ihrer verschiedenen Löslichkeiten, in die Diastereomeren, aus denen das gewünschte Enantiomere durch Einwirkung geeigneter Mittel freigesetzt werden kann. Vorteilhaft isoliert man das wirksamere Enantiomere.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Derivates bzw. Salzes und/oder seiner Racemate bzw. Antipoden verwendet oder insbesondere unter den Reaktionsbedingungen bildet.

Beim Verfahren der vorliegenden Erfindung werden vorzugsweise solche Ausgangsstoffe verwendet, welche zuden eingangs als besonders wertvoll geschilderten Verbindungen I führen. Neue Ausgangsstoffe, die speziell für die Herstellung der erfindungsgemässen Verbindungen entwickelt wurden, ihre Verwendung und Verfahren zu ihrer Herstellung bilden ebenfalls einen Gegenstand der Erfindung, wobei die Variablen $R_1$, $R_2$, $R_3$, $R_4$, $R_5$ und n die für die jeweils bevorzugten Verbindungsgruppen der Formel I bzw. Tautomere davon angegebenen Bedeutungen haben. Insbesondere sind Verbindungen der Formel III, ihre Tautomeren und Salze, worin $X_3$ Amino bedeutet, als Ausgangsmaterial bevorzugt (Verbindungen III').

Die Erfindung betrifft ebenfalls die Verwendung der Verbindungen der Formel I bzw. ihrer Tautomeren oder von pharmazeutisch verwendbaren Salzen von solchen Verbindungen mit salzbildenden Eigenschaften, insbesondere als pharmakologische, in erster Linie nootrope, antidepressive und antiparkinson-wirksame, Wirksubstanzen. Dabei kann man sie, vorzugsweise in Form von pharmazeutisch verwendbaren Zubereitungen, in einem Verfahren zur prophylaktischen und/oder therapeutischen Behandlung des tierischen oder menschlichen Körpers, insbesondere als Nootropika, Antidepressiva und Mittel zur Behandlung des Parkinson-Syndroms, verwenden.

Die Erfindung betrifft gleichfalls pharmazeutische Präparate, die die erfindungsgemässen Verbindungen

oder pharmazeutisch verwendbare Salze derselben als Wirkstoffe enthalten, sowie Verfahren zu ihrer Herstellung.

Bei den erfindungsgemässen pharmazeutischen Präparaten, welche die erfindungsgemässe Verbindung oder pharmazeutisch verwendbare Salze davon enthalten, handelt es sich um solche zur enteralen, wie oralen, ferner rektalen, und parenteralen Verabreichung an Warmblüter, wobei der pharmakologische Wirkstoff allein oder zusammen mit einem pharmazeutisch anwendbaren Trägermaterial enthalten ist.

Die neuen pharmazeutischen Präparate enthalten z.B. von etwa 10 % bis etwa 80 %, vorzugsweise von etwa 20% bis etwa 60%, des Wirkstoffs. Erfindungsgemässe pharmazeutische Präparate zur enteralen bzw. parenteralen Verabreichung sind z.B. solche in Dosiseinheitsformen, wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferne Bindemittel, wie Stärkekleister, unter Verwendung z.B. von Mais-, Weizen-, Reis- oder Kartoffelstärke, Gelatine, Tragakanth, Methylcellulose und/oder Polyvinylpyrrolidon, wenn erwünscht, Sprengmittel, wie die obengenannten Stärken, ferner Carboxmethylstärke, quervernetzes Polyvinylpyrrolidon, Agar, Alginsäure oder ein Salz davon, wie Natriumalginat, Hilfsmittel sind in erster Linie Fliess-, Fliessregulier- und Schmiermittel, z.B. Kieselsäure, Talk, Stearinsäure oder Salze davon, wie Magnesium- oder Calciumstearat, und/oder Polyethylenglykol. Dragée-Kerne werden mit geeigneten, gegebenenfalls Magensaftresistenten Ueberzügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemische oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls Stabilisatoren, enthalten. In weichen Kapseln ist der Wirkstoff vorzugsweise in geeigneten Flüssigkeiten, wie fetten Oelen, Paraffinöl oder flüssigen Polyethylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositorienmasse eignen sich z.B. natürliche oder synthetische Triglyceride, Paraffinkohlenwasserstoffe, Polyethylenglykole oder höhere Alkanole. Ferner können auch Gelatine-Rektalkapseln verwendet werden, die eine Kombination des Wirkstoffs mit einem Grundmassenstoff enthalten. Als Grundmassenstoffe kommen z.B. flüssige Triglyceride, Polyethylenglykole oder Paraffinkohlenwasserstoffe in Frage.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z.B. Sesamöl,oder synthetische Fettsäureester, z.B. Ethyloleat oder Triglyceride, verwendet, oder wässrige Injektionssuspensionen, welche viskositätserhöhende Stoffe, z.B. Natrium-carboxymethylcellulose, Sorbit und/oder Dextran, und gegebenenfalls auch Stabilisatoren enthalten.

Die Dosierung des Wirkstoffs hängt von der Warmblüter-Spezies, dem Alter und dem individuellen Zustand sowie der Applikationsweise ab. Im Normalfall ist für einen etwa 75 kg schweren Warmblüter bei oraler Applikation eine ungefähre Tagesdosis von etwa 150 mg bis etwa 1500 mg, vorteilhaft in mehreren gleichen Teildosen, zu veranschlagen.

Die nachfolgenden Beispiele illustrieren die oben beschriebene Erfindung; sie sollen jedoch diese in ihrem Umfang in keiner Weise einschränken. Temperaturen sind in Celsius graden angegeben.

Beispiel 1: 8,3 g (76 mMol) 6-Amino-2-hydroxypyridin und 20g (114 mMol) N,N-Di-n-propylformamiddimethylacetal werden in 70 ml Xylol unter Argon vorgelegt. Unter Rühren wird eine $\frac{1}{2}$ halbe Stunde auf 100° erhitzt. Dann lässt man abkühlen, dampft im Wasserstrahlvakuum ein und filtriert den Rückstand über

die zehnfache Menge Florisil mittels Methylenchlorid. Die das Produkt enthaltenden Fraktionen werden eingedampft und anschliessend aus n-Hexan kristallisiert. Man erhält N-(2-Hydroxy-6-pyridyl)-N',N'-di-n-propyl-formamidin bzw. N-(2-Pyridon-6-yl)-N',N'-di-n-propylformamidin vom Smp. 102-104°.

Zur Ueberführung ins Methansulfonat löst man die freie Base in Methylenchlorid und versetzt dann mit Methansulfonsäure, bis ein pH von 3 erreicht wird. Dann wird unter Rühren Ether zugegeben, wobei das Produkt spontan auskristallisiert. Die Kristalle werden abgenutscht, mit Ether gut nach gewaschen und im Hochvakuum getrocknet. Man erhält so N-(2-Hydroxy-6-pyridyl)-N',N'-di-n-propyl-formamidinmethansulfonat

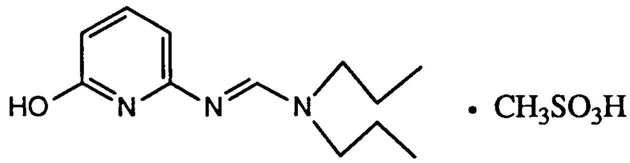

bzw. N-(2-Pyridon-6-yl)-N',N'-di-n-propyl-formamidin-methansulfonat

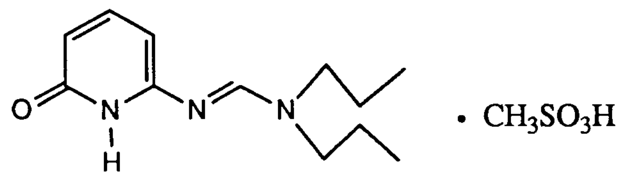

vom Smp. 160-162°.

Beispiel 2: In Analogie zu Beispiel 1 kann N-(2-Hydroxy-6-pyridyl)-N',N'-dimethylformamidin bzw. N-(2-Pyridon-6-yl)-N',N'-dimethylformamidin vom Smp. 159-161° hergestellt werden; ausgehend von 3,3 g (30 mMol) 6-Amino-2-hydroxypyridin und 5,4 g (45 mMol) N,N-Dimethylformamid-dimethylacetalin 30 ml Xylol.

Beispiel 3: Analog zu Beispiel 1 kann N-(2-Hydroxy-6-pyridyl)-N'-methyl-N'-butylformamidin bzw. N-(2-Pyridon-6-yl)-N'-methyl-N'-butyl-formamidin vom Smp. 85-86° erhalten werden; ausgehend von 3 g (27 mMol) 6-Amino-2-hydroxypyridin und 6,6 g (41 mMol) N-Methyl-N-butyl-formamid-dimethylacetalin 30ml Toluol.

Beispiel 4: Analog zu Beispiel 1 erhält man N-(2-Hydroxy-6-pyridyl)-N'-methyl-N'-(2-phenylethyl)-acetamidin-dihydrochlorid bzw. N-(2-Pyridon-6-yl)-N'-methyl-N'-(2-phenylethyl)-acetamidin-di hydrochlorid vom Smp. 177-179°; ausgehend von 4 g (24 mMol) N-(2-Hydroxy-6-pyridyl)-acetimidsäuremethylester und 4,9 g (36 mMol) Methylphenylethylamin in 20 ml Xylol.

Das Ausgangsmaterial kann wie folgt hergestellt werden: 4,4 g (40 mMol) 6-Amino-2-hydroxypyridin werden in 30 ml Orthoessigsäuretrimethylester während 12 Stunden zum Rückfluss erhitzt. Dann wird das Reaktionsgemisch eingedampft. Der Rückstand wird mit Ether unter Rühren versetzt, wobei das Produkt spontan auskristallisiert. Es wird abgenutscht und im Hochvakuum getrocknet. Man erhält so N-(2-Hydroxy-6-pyridyl)-acetimidsäuremethylester vom Smp. 128-129°.

Beispiel 5: In Analogie zu Beispiel 4 kann N-(2-Hydroxy-6-pyridyl)-N',N'-di-n-propyl-acetamidin bzw. N-(2-Pyridon-6-yl)-N',N'-di-n-propyl-acetamidin vom Smp. 140-141° erhalten werden; ausgehend von 2 g (12 mMol) N-(2-Hydroxy-6-pyridyl)-acetimidsäuremethylester und 1,8 g (18 mMol) Di-n-propylamin in 20 ml Xylol.

Beispiel 6: Zu einer Suspension von 1,1 g (22,4 mMol) NaH (50 % Dispersion in Mineralöl) in 30 ml abs. Tetrahydrofuran wird bei Raumtemperatur unter Argon und unter Rühren eine Lösung von 4,5 g (20,3 mMol) N-(2-Hydroxy-6-pyridyl)-N',N'-di-n-propylformamidin in 15 ml abs. Tetrahydrofuran getropft. Man rührt weitere 15 Minuten bei dieser Temperatur und gibt dann eine Lösung m von 1,4 ml (22,4 mMol) Methyliodid in 5 ml Tetrahydrofuran zu. Dieses Reaktionsgemisch wird 24 Stunden bei Raumtemperatur gerührt. Dann wird mit Essigsäureethylester verdünnt und einmal mit Wasser gewaschen. Die organische Phase wird abgetrennt, getrocknet und eingedampft. Das so erhaltene Rohprodukt wird dann an Kieselgel chromatographisch gereinigt. Das gereinigte Produkt wird mit einem halben Aequivalent Fumarsäure in Ether versetzt und durch nach folgende Zugabe von Petrolether auskristallisiert. Man erhält N-(1-Methyl-2-pyridon-6-yl)-N',N'-di-n-propylformamidin-hemif umarat vom Smp. 92-94°.

Beispiel 7: Zu einer Lösung von 2,7 g (10 mMol) N-(2-Methoxy-6-pyridyl)-N',N'-di-n-propylformamidinhydrochlorid in Methylenchlorid wird bei -60° C unter Rühren 1,2 g (10 mMol) $BCl_3$ langsam zudosiert. Dann wird noch 30 Minuten bei dieser Temperatur verrührt. Man lässt darauf auf 0° C erwärmen und versetzt nach einer weiteren Stunde vorsichtig mit 15 ml absolutem Methanol. Das Reaktionsgemisch wird dann auf Eiswasser gegossen, mit 2 N Natronlauge alkalisch gestellt und mit Methylenchlorid zweimal extrahiert. Die organischen Phasen werden vereinigt, über $Na_2SO_4$ getrocknet, über eine Florisilschicht filtriert und dann eingedampft. Das so erhaltene Rohprodukt wird aus Ether/Petrolether kristallisiert. Man erhält N-(2-Hydroxy-6-pyridyl)-N',N'-di-n-propylformamidin bzw. N-(2-Pyridon-6-yl)-N',N'-di-n-propylformamidin vom Smp. 102-104°.

Das Ausgangsmaterial N-(2-Methoxy-6-pyridyl)-N',N'-di-n-propylformamidin-hydrochlorid vom Smp. 148-149° C kann in Analogie zu Beispiel 1 hergestellt werden; ausgehend von 1,24 g (10 mMol) 2-Amino-6-methoxypyridin und 2,63 g (15 mMol) N,N-Di-n-propylformamid-dimethylacetal in 20 ml Xylol.

Beispiel 8: In analoger Weise wie in einem der Beispiele 1-7 beschrieben kann man herstellen:
N-(1-Methyl-2-pyridon-6-yl)-N',N'-dipropyl-acetamidin,
N-(1-Benzyl-2-pyridon-6-yl)-N',N'-dipropyl-formamidin,
N-(2-Pyridon-6-yl)-N'-propyl-acetamidin bzw. N-(2-Hydroxy-6-pyridyl)-N'-propyl-acetamidin,
N-(2-Pyridon-6-yl)-N'-ethyl-N'-isopropyl-formamidin bzw. N-(2-Hydroxy-6-pyridyl)-N'-ethyl-N'-isopropyl-formamidin,
N-(2-Pyridon-6-yl)-N',N'-dipropyl-propionamidin bzw. N-(2-Hydroxy-6-pyridyl)-N',N'-dipropyl-propionamidin,
N-(1-Propyl-2-pyridon-6-yl)-N',N'-dipropyl-formamidin.

Beispiel 9: In Analogie zu Beispiel 6 kann man N-(1-Propyl-2-pyridon-6-yl)-N',N'-di-n-propyl-formamidin herstellen, ein viskoses Oel mit Rf = 0,43 (Toluol/Ethanol/konz. wässriges $NH_3$ = 90:20:1).

Man geht aus von 3 g (14 mMol) N-(2-Hydroxy-6-pyridyl)-N',N'-di-n-propylformamidin bzw. N-(2-Pyridon-6-yl)-N',N'-di-n-propylformamidin, 710 mg (15 mMol) NaH als 50 % Dispersion in Mineralöl und 1,5 ml (15 mMol) n-Propyljodid in 30 ml absolutem Tetrahydrofuran. Im Unterschied zu Beispiel 6 lässt man 56 Stunden unter Rückfluss reagieren.

Beispiel 10: In analoger Weise wie in Beispiel 6 beschrieben kann N-(1-Methyl-2-pyridon-6-yl)-N',N'-di-n-propyl-acetamidin hergestellt werden. Es wird als viskoses Oel erhalten, Rf = 0,28 (Methylenchlorid/Methanol/konz. wässriges $NH_3$ = 300:10:1).

Ausgegangen wird von 1 g (4,4 mMol) N-(2-Hydroxy-6-pyridyl)-N',N'-di-n-propylacetamidin bzw. N-(2-Pyridon-6-yl)-N',N'-di-n-propylacetamidin, 230 mg (4,6 mMol) NaH (50 % Dispersion) und 290 µl (4,6 mMol) Methyljodid in 10 ml absolutem Tetrahydrofuran.

Beispiel 11: In Analogie zu Beispiel 6 kann N-(1-Benzyl-2-pyridon-6-yl)-N',N'-di-n-propylformamidin als viskoses Oel, Rf = 0,35 (Hexan/Essigsäureethylester = 1:4), hergestellt werden. Man geht aus von 3 g (14 mMol) N-(2-Hydroxy-6-pyridyl)-N',N'-di-n-propylformamidin bzw. N-(2-Pyridon-6-yl)-N',N'-di-n-propyl-formamidin, 720 mg (15 mMol) NaH (50 % Dispersion) und 1,8 ml (15 mMol) Benzylbromid in 30 ml absolutem Tetrahydrofuran.

Beispiel 12: Zu einer Lösung von 9,1 g (58 mMol) N,N-Di-n-propylpropionamid in 90 ml absolutem Chloroform werden bei 0° unter Rühren und Feuchtigkeitsausschluss 37 ml einer 1,9 molaren Lösung von Phosgen in Toluol zugetropft. Dieses Gemisch wird 5 Stunden bei 0° gerührt und dann im Vakuum eingeengt. Der Rückstand wird in 40 ml absolutem Chloroform aufgenommen und zu einer Suspension von 6,4 g (58 mMol) 2-Amino-6-hydroxypyridin in 50 ml absolutem Chloroform getropft. Anschliessend wird noch mit 20 ml (145 mMol) Triethylamin versetzt und 20 Stunden bei Raumtemperatur weitergerührt. Das Reaktionsgemisch wird dann mit Dichlormethan verdünnt und zweimal mit Wasser gewaschen. Die organische Phase wird über $MgSO_4$ getrocknet und dann im Vakuum eingeengt. Das resultierende Rohprodukt wird an Kieselgel chromatographisch gereinigt und darauf aus Ether/Petrolether kristallisiert. Man erhält N-(2-Pyridon-6-yl)-N',N'-di-n-propyl-propionamidin bzw. N-(2-Hydroxy-6-pyridyl)-N',N'-di-n-propyl-propionamidin vom Smp. 112-113°.

Beispiel 13: In Analogie zu Beispiel 12 kann man N-(2-Pyridon-6-yl)-N'-ethyl-N'-isopropyl-acetamidin bzw. N-(2-Hydroxy-6-pyridyl)-N'-ethyl-N'-isopropyl-acetamidin vom Smp. 110-111° herstellen; ausgehend von 8,4g (65 mMol) N-Ethyl-N-isopropylacetamid, 40 ml einer 1,9-molaren Lösung von Phosgen in Toluol, 7,2g (65 mMol) 2-Amino-6-hydroxypyridin und 16,5 g (163 mMol) Triethylamin.

Das Ausgangsmaterial kann folgendermassen hergestellt werden: 8,7 g (100 mMol) N-Ethyl-N-isopropylamin werden mit 50 ml Essigsäureanhydrid vorsichtig vermischt und 1 Stunde unter Rückfluss erwärmt. Dann wird im Wasserstrahlvakuum eingeengt. Der Rückstand wird mit Dichlormethan aufgenommen, je einmal mit 2N Salzsäure, 2N Natronlauge und Wasser gewaschen. Die organische Phase wird getrocknet und eingeengt. Das zurückbleibende Oel wird im Hochvakuum destilliert. Man erhält N-Ethyl-N-isopropylacetamid vom Sdp. 60-62°/0,08 mm/Hg.

Beispiel 14: Zu einer gerührten Lösung von 8,3 ml (99 mMol) n-Propylamin in 8 ml Wasser wird bei Raumtemperatur im Zeitraum von 20 Minuten portionenweise 2,3 g (14 mMol) N-(2-Hydroxy-6-pyridyl)-N'-cyano-formamidin zugegeben. Das Reaktionsgemisch wird 2 Stunden bei Raumtemperatur gerührt. Dann wird dreimal mit Chloroform extrahiert. Die vereinigten organischen Phasen werden über $MgSO_4$ getrocknet und eingedampft. Das so erhaltene Rohprodukt wird an Kieselgel mit Methylenchlorid chromatographiert und anschliessend aus Ether/n-Hexan kristallisiert. Man erhält N-(2-Pyridon-6-yl)-N'-propyl-formamidin bzw. N-(2-Hydroxy-6-pyridyl)-N'-propylformamidin vom Smp. 178-179°.

Das Ausgangsmaterial kann folgendermassen hergestellt werden: 11 g (100 mMol) 2-Amino-6-hydroxypyridin und 9,8 g (100 mMol) Ethyl-N-cyanoformamidin werden in 100 ml Ethanol 12 Stunden unter Rückfluss gerührt. Das Reaktionsgemisch wird dann im Vakuum eingeengt und an Kieselgel chromatographisch gereinigt. Das so gereinigte Produkt wird in Chloroform/Methanol gelöst und durch Zugabe von Ether auskristallisiert. Man erhält N-(2-Hydroxy-6-pyridyl)-N'-cyano-formamidin bzw. N-(2-Pyridon-6-yl)-N'-cyano-formamidin vom Smp. 216-218°.

Beispiel 15: Analog wie in Beispiel 14 beschrieben kann man N-(2-Hydroxy-6-pyridyl)-N',N'-di-n-propyl-formamidin bzw. N-(2-Pyridon-6-yl)-N',N'-di-n-propyl-formamidin vom Smp. 102-104° herstellen; ausgehend von 13,7 ml (100 mMol) Di-n-propylamin und 2,3 g (14 mMol) N-(2-Hydroxy-6-pyridyl)-N'-cyano-formamidin in 10 ml Wasser.

Beispiel 16: In Analogie zu Beispiel 12 kann N-(2-Hydroxy-6-pyridyl)-N',N'-di-n-propyl-formamidin bzw. N-(2-Pyridon-6-yl)-N',N'-di-n-propyl-formamidin vom Smp. 102-104° erhalten werden; ausgehend von 6,5 g (50 mMol) N,N-Di-n-propylformamid, 35ml einer 1,9-molaren Lösung von Phosgen in Toluol, 5,5 g (50 mMol) 2-Amino-6-hydroxypyridin und 12,7 g (125 mMol) Triethylamin.

Beispiel 17: 4,25 g (50 mMol) N-Cyano-acetamidin und 6,9 g (50 mMol) N,N-Dipropylamin-hydrochlorid werden in 40 ml Wasser unter Rühren für 3 Stunden auf 100° erwärmt. Das abgekühlte Reaktionsgemisch wird mit 2N Natronlauge alkalisch gestellt, worauf sich ein Oel abscheidet. Dies wird abgetrennt und die wässrige Phase noch einmal mit Dichlormethan extrahiert. Die organischen Phasen werden vereinigt und eingeengt. Der Rückstand wird in Dioxan aufgenommen und zu einer Suspension von 4,9 g (44 mMol) 2-Amino-6-hydroxy-pyridin in 20 ml Xylol gegeben und 12 Stunden unter Rückfluss erwärmt. Das Reaktionsgemisch wird eingeengt und über Kieselgel chromatographisch gereinigt. Das so gereinigte Produkt wird aus Chloroform/Ether kristallisiert. Man erhält N-(2-Hydroxy-6-pyridyl)-N',N'-di-n-propyl-acetamidin bzw. N-(2-Pyridon-6-yl)-N',N'-di-n-propyl-acetamidin vom Smp. 140-141°.

Beispiel 18: 3,3 g (20 mMol) N-(2-Hydroxy-6-pyridyl)-N',N'-dimethyl-formamidin bzw. N-(2-Pyridon-6-yl)-N',N'-dimethyl-formamidin und 5,1 g (50 mMol) N,N-Di-n-propylamin werden in 20 ml Xylol 12 Stunden unter Rückfluss gerührt. Das Reaktionsgemisch wird eingedampft und dann über die zehnfache Menge Florisil mittels Dichlormethan filtriert. Die produktehaltigen Fraktionen werden vereinigt und eingedampft. Kristallisation aus Dichlormethan/n-Hexan ergibt N-(2-Hydroxy-6-pyridyl)-N',N'-di-n-propylformamidin bzw. N-(2-Pyridon-6-yl)-N',N'-di-n-propylformamidin vom Smp. 102-104°.

Beispiel 19: 1,8 g (10 mMol) N-(2-Pyridon-6-yl)-N'-propyl-formamidin bzw. N-(2-Hydroxy-6-pyridyl)-N'-propyl-formamidin, 1,9 g (11 mMol) Propyljodid und 1,5 g (11 mMol) Kaliumcarbonat werden in 30 ml absolutem Ethanol bei 80°C 24 Stunden verrührt. Dann wird das Reaktionsgemisch filtriert und eingedampft. Der Rückstand wird in Methylenchlorid aufgenommen, mit Wasser gewaschen, dann über $Na_2SO_4$ getrocknet und eingedampft. Das so erhaltene Rohprodukt wird chromatographisch gereinigt. Kristallisation aus Methylenchlorid/Ether liefert N-(2-Pyridon-6-yl)-N',N'-di-n-propyl-formamidin bzw. N-(2-Hydroxy-6-pyridyl)-N',N'-di-n-propyl-formamidin vom Smp. 102-104°.

Beispiel 20: Analog wie in Beispiel 14 beschrieben kann man N-(2-Hydroxy-6-pyridyl)-formamidin bzw. N-(2-Pyridon-6-yl)-formamidin herstellen; ausgehend von 1,8 g (10 mMol) N-(2-Hydroxy-6-pyridyl)-N'-cyano-formamidin und 40 ml Ammoniak gesättigtes Ethanol bei 60°.

Beispiel 21: Analog wie in einem der Beispiele 1-20 beschrieben kann man herstellen:

N-(2-Hydroxy-3-methyl-6-pyridyl)-N',N'-di-n-propyl-formamidin bzw. N-(3-Methyl-2-pyridon-6-yl)-N',N'-di-n-propyl-formamidin,

N-(5-Trifluormethyl-2-hydroxy-6-pyridyl)-N',N'-di-n-propyl-formamidin bzw. N-(5-Trifluormethyl-2-pyridon-6-yl)-N',N'-di-n-propylformamidin,

N-(4-Chlor-2-hydroxy-6-pyridyl)-N',N'-di-n-propyl-formamidin bzw. N-(4-Chlor-2-pyridon-6-yl)-N',N'-di-n-propyl-formamidin.

Beispiel 22: Tabletten, enthaltend je 50 mg des Wirkstoffs, z.B. N-(2-Hydroxy-6-pyridyl)-N',N'-di-n-propyl-formamidin-methansulfonat bzw. N-(2-Pyridon-6-yl)-N',N'-di-n-propyl-formamidin-methansulfonat, können wie folgt hergestellt werden.

| Zusammensetzung (10000 Tabletten) | |
|---|---|
| Wirkstoff | 500.0 g |
| Lactose | 500.0 g |
| Kartoffelstärke | 352.0 g |
| Gelatine | 8.0 g |
| Talk | 60.0 g |
| Magnesiumstearat | 10.0 g |
| Siliciumdioxid (hochdisper.) | 20.0 g |
| Ethanol | q.s. |

Der Wirkstoff wird mit der Lactose und 292 g Kartoffelstärke vermischt, die Mischung mit einer alkoholischen Lösung der Gelatine befeuchtet und durch ein Sieb granuliert. Nach dem Trocknen mischt man den Rest der Kartoffelstärke, den Talk, das Magnesiumstearat und das hochdisperse Siliciumdioxid zu und presst das Gemisch zu Tabletten von je 145,0 mg Gewicht und 50,0 mg Wirkstoffgehalt, die gewünschtenfalls mit Teilkerben zur feineren Anpassung der Dosierung versehen sein können.

Beispiel 23: Lacktabletten, enthaltend je 100 mg des Wirkstoff, z.B. N-(2-Hydroxy-6-pyridyl)-N',N'-di-n-propyl-formamidin-methansulfonat bzw. N-(2-Pyridon-6-yl)-N',N'-di-n-propyl-formamidin-methansulfonat,können wie folgt hergestellt werden:

| Zusammensetzung (für 1000 Tabletten) | |
|---|---|
| Wirkstoff | 100.00 g |
| Lactose | 100.00 g |
| Maisstärke | 70.00 g |
| Talk | 8.50 g |
| Calciumstearat | 1.50 g |
| Hydroxypropyl-methylcellulose | 2.36 g |
| Schellack | 0.64 g |
| Wasser | q.s. |
| Methylenchlorid | q.s. |

Der Wirkstoff, die Lactose und 40 g der Maisstärke werden gemischt und mit einem Kleister, hergestellt aus 15 g Malsstärke und Wasser (unter Erwärmen) befeuchtet und granuliert. Das Granulat wird getrocknet, der Rest der Maisstärke, der Talk und das Calciumstearat werden zugegeben und mit dem Granulat vermischt. Das Gemisch wird zu Tabletten (Gewicht: 280 mg) verpresst und diese mit einer Lösung der Hydroxypropylmethylcellulose und des Schellacks in Methylenchlorid lackiert; Endgewicht der Lacktablette: 283 mg.

Beispiel 24: In analoger Weise wie in den Beispielen 22 und 23 beschrieben können auch Tabletten bzw. Lacktabletten, enthaltend eine erfindungsgemässe Verbindung, z.B. gemäss den Beispielen 1 bis 19 und 21, hergestellt werden.

**Patentansprüche**

**Patentansprüche für folgende Vertragsstaaten : BE, CH, DE, FR, GB, IT, LI, LU, NL, SE**

**1.** Pyridin-Derivate der Formel

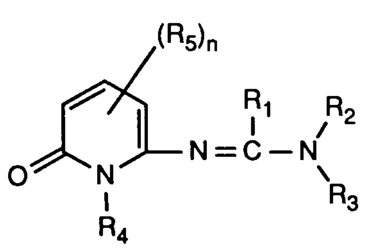

(I)

und ihre Tautomeren und/oder ihre Salze, worin $R_1$ Wasserstoff oder $C_1$-$C_7$-Alkyl bedeutet, einer der Reste $R_2$ und $R_3$ Wasserstoff, $C_1$-$C_7$-Alkyl, Phenyl- oder Naphthyl-$C_1$-$C_7$-alkyl, $C_3$-$C_6$-Alkenyl oder $C_3$-$C_6$-Alkinyl und der andere $C_1$-$C_7$-Alkyl, Phenyl- oder Naphthyl-$C_1$-$C_7$-alkyl, $C_3$-$C_6$-Alkenyl oder $C_3$-$C_6$-Alkinyl bedeutet, $R_4$ Wasserstoff, $C_1$-$C_7$-Alkyl oder Phenyl- oder Naphthyl-$C_1$-$C_7$-alkyl bedeutet, $R_5$ $C_1$-$C_7$-Alkyl oder Trifluormethyl bedeutet und der Index n 0, 1 oder 2 bedeutet.

**2.** Verbindungen gemäss Anspruch 1 der Formel I und ihre Tautomeren und/oder ihre Salze, worin $R_1$ Wasserstoff oder $C_1$-$C_7$-Alkyl bedeutet, einer der Reste $R_2$ und $R_3$ Wasserstoff, $C_1$-$C_7$-Alkyl oder Phenyl- oder Naphthyl-$C_1$-$C_7$-alkyl und der andere $C_1$-$C_7$-Alkyl oder Phenyl- oder Naphthyl-$C_1$-$C_7$-alkyl bedeutet, $R_4$ Wasserstoff oder $C_1$-$C_7$-Alkyl bedeutet, $R_5$ $C_1$-$C_7$-Alkyl bedeutet und der Index n 0 oder 1 ist.

**3.** Verbindungen gemäss Anspruch 1 der Formel I und ihre Tautomeren und/oder ihre Salze, worin $R_1$ Wasserstoff oder $C_1$-$C_7$-Alkyl bedeutet, einer der Reste $R_2$ und $R_3$ Wasserstoff, $C_1$-$C_7$-Alkyl oder Phenyl- oder Naphthyl-$C_1$-$C_7$-alkyl und der andere $C_1$-$C_7$-Alkyl oder Phenyl- oder Naphthyl-$C_1$-$C_7$-alkyl bedeutet, $R_4$ Wasserstoff bedeutet und der Index n 0 ist.

**4.** Verbindungen gemäss Anspruch 1, 2 oder 3 der Formel I und ihre Tautomeren und/oder ihre Salze, worin einer der Reste $R_2$ und $R_3$ Wasserstoff, $C_1$-$C_7$-Alkyl oder Phenyl- oder Naphthyl-$C_1$-$C_7$-alkyl und der andere $C_1$-$C_7$-Alkyl oder Phenyl- oder Naphthyl-$C_1$-$C_7$-alkyl bedeutet.

**5.** Verbindungen gemäss Anspruch 3 der Formel I und ihre Tautomeren und/oder ihre Salze, worin $R_1$ Wasserstoff oder $C_1$-$C_4$-Alkyl bedeutet, $R_2$ und $R_3$ unabhängig voneinander $C_1$-$C_4$-Alkyl bedeuten oder $R_2$ $C_1$-$C_4$-Alkyl bedeutet und $R_3$ Phenyl-$C_1$-$C_4$-alkyl bedeutet, $R_4$ Wasserstoff ist und der Index n 0 ist.

**6.** Verbindungen gemäss Anspruch 2 der Formel I und ihre Tautomeren und/oder ihre Salze, worin $R_1$ Wasserstoff oder $C_1$-$C_4$-Alkyl bedeutet, $R_2$ und $R_3$ unabhängig voneinander $C_1$-$C_4$-Alkyl bedeuten oder $R_2$ $C_1$-$C_4$-Alkyl bedeutet und $R_3$ Phenyl-$C_1$-$C_4$-alkyl bedeutet, $R_4$ $C_1$-$C_4$-Alkyl ist und der Index n 0 ist.

**7.** Verbindungen gemäss Anspruch 3 der Formel I und ihre Tautomeren und/oder ihre Salze, worin $R_1$ und $R_4$ Wasserstoff bedeuten, $R_2$ und $R_3$ unabhängig voneinander $C_1$-$C_4$-Alkyl bedeuten und der Index n 0 ist.

**8.** Verbindungen gemäss Anspruch 2 der Formel I und ihre Tautomeren und/oder ihre Salze, worin $R_1$ Wasserstoff bedeutet, $R_4$ $C_1$-$C_4$-Alkyl bedeutet, $R_2$ und $R_3$ unabhängig voneinander $C_1$-$C_4$-Alkyl bedeuten und der Index n 0 ist.

**9.** Verbindungen gemäss Anspruch 2 der Formel I und ihre Tautomeren und/oder ihre Salze, worin $R_1$ Methyl ist, $R_4$ Wasserstoff bedeutet, $R_2$ und $R_3$ unabhängig voneinander $C_1$-$C_4$-Alkyl bedeuten und der Index n 0 ist.

**10.** Verbindungen gemäss Anspruch 5 der Formel I und ihre Tautomeren und/oder ihre Salze, worin $R_1$ Wasserstoff oder Methyl bedeutet, $R_2$ Methyl bedeutet, $R_3$ 2-Phenylethyl bedeutet, $R_4$ Wasserstoff ist und der Index n 0 ist.

**11.** Verbindungen gemäss Anspruch 6 der Formel I und ihre Tautomeren und/oder ihre Salze, worin $R_1$ Wasserstoff oder Methyl bedeutet, $R_2$ Methyl bedeutet, $R_3$ 2-Phenylethyl bedeutet, $R_4$ Wasserstoff ist und der Index n 0 ist.

**12.** N-(2-Hydroxy-6-pyridyl)-N',N'-di-n-propyl-formamidin bzw. N-(2-Pyridon-6-yl)-N',N'-di-n-propyl-formamidin oder ein Salz davon.

**13.** N-(2-Hydroxy-6-pyridyl)-N',N'-di-n-propyl-acetamidin bzw. N-(2-Pyridon-6-yl)-N',N'-di-n-propyl-acetamidin oder ein Salz davon.

**14.** N-(2-Hydroxy-6-pyridyl)-N'-methyl-N'-(2-phenylethyl)-acetamid in bzw. N-(2-Pyridon-6-yl)-N'-methyl-N'-(2-phenylethyl)-acetamidin oder ein Salz davon.

**15.** N-(1-Methyl-2-pyridon-6-yl)-N',N'-di-n-propylformamidin oder ein Salz davon.

**16.** N-(1-Methyl-2-pyridon-6-yl)-N',N'-dipropyl-acetamidin oder ein Salz davon.

**17.** N-(1-Benzyl-2-pyridon-6-yl)-N',N'-dipropyl-formamidin oder ein Salz davon.

**18.** Eine Verbindung gemäss einem der Ansprüche 1-17 oder ein Tautomeres und/oder ein pharmazeutisch verwendbares Salz davon zur Anwendung in einem Verfahren zur therapeutischen oder prophylaktischen Behandlung des menschlichen oder tierischen Körpers.

**19.** Eine Verbindung gemäss einem der Ansprüche 1-18 oder ein Tautomeres und/oder ein pharmazeutisch verwendbares Salz davon zur Anwendung als Nootropikum, Antidepressivum und Antiparkinson-Mittel.

**20.** Pharmazeutische Präparate enthaltend eine Verbindung oder ein Tautomeres gemäss einem der Ansprüche 1-19 in freier Form oder in Form eines pharmazeutisch verwendbaren Salzes.

**21.** Nootropes, antidepressives und antiparkinsonaktives pharmazeutisches Präparat gemäss Anspruch 20.

**22.** Verwendung einer Verbindung gemäss einem der Ansprüche 1-19 oder eines Tautomeren und/oder eines pharmazeutisch verwendbaren Salzes davon zur Herstellung von Nootropika, Antidepressiva und Antiparkinson-Mitteln.

**23.** Verfahren zur Herstellung von Verbindungen oder Tautomeren und/oder Salzen gemäss einem der Ansprüche 1-17, dadurch gekennzeichnet, dass man
a) eine Verbindung der Formel

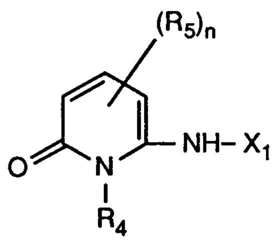

(IIa)

oder ein Tautomeres davon mit einer Verbindung der Formel

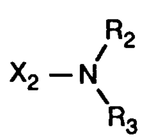

(IIb),

worin einer der Reste $X_1$ und $X_2$ für eine Gruppe der Formel $-CO-R_1$ steht und der andere Wasserstoff oder eine Gruppe der Formel $-CO-Z_1$ ist, worin $Z_1$ einen abspaltbaren Rest bedeutet, oder einem Tautomeren, Salz und/oder Acetal davon umsetzt oder
b) in einer Verbindung der Formel

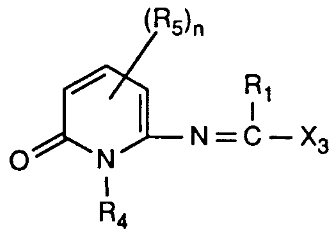

(III)

oder einem Tautomeren und/oder Salz davon, worin $X_3$ einen in

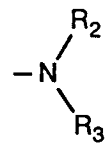

überführbaren Rest bedeutet, $X_3$ in

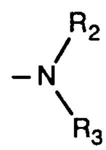

überführt oder

c) eine Verbindung der Formel

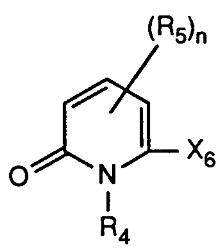

(Va)

oder ein Tautomeres oder Salz davon mit einer Verbindung der Formel

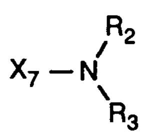

(Vb)

oder einem Tautomeren oder einem Salz davon, wobei $X_6$ für die Gruppe $-N=CR_1-NH-X_6$ steht und $X_7$ für Wasserstoff steht oder $X_6$ für $-NH_2$ steht und $X_7$ für die Gruppe $-CR_1=N-X_6$ steht und $X_6$ für eine Abgangsgruppe steht, umsetzt

und gewünschtenfalls jeweils eine verfahrensgemäss oder auf andere Weise erhältliche Verbindung der Formel I oder ein Tautomeres davon in eine andere Verbindung der Formel I bzw. ein Tautomeres davon überführt, ein verfahrensgemäss erhältliches Isomerengemisch in die Komponenten auftrennt und/oder eine verfahrensgemäss erhältliche freie Verbindung der Formel I bzw. ein Tautomeres davon in ein Salz überführt oder ein verfahrensgemäss erhältliches Salz in die freie Verbindung der Formel I

bzw. ein Tautomeres davon oder in ein anderes Salz überführt.

**24.** Verfahren zur Herstellung von Verbindungen oder Tautomeren und/oder Salzen gemäss einem der Ansprüche 1 bis 5, 7, 9, 10 und 12 bis 14 der Formel I, worin $R_4$ Wasserstoff bedeutet, dadurch gekennzeichnet, dass man in einer Verbindung der Formel

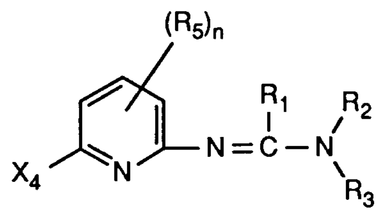

(IV)

oder einem Salz davon, worin $X_4$ für geschütztes Hydroxy steht, die Hydroxyschutzgruppe abspaltet und gewünschtenfälls eine verfahrensgemäss oder auf andere Weise erhältliche Verbindung der Formel I oder ein Tautomeres davon in eine andere Verbindung der Formel I bzw. ein Tautomeres davon überführt,ein verfahrensgemäss erhältliches Isomerengemisch in die Komponenten auftrennt und/oder eine verfahrensgemäss erhältliche freie Verbindung der Formel I bzw. ein Tautomeres davon in ein Salz überführt oder ein verfahrensgemäss erhältliches Salz in die freie Verbindung der Formel I bzw. ein Tautomeres davon oder in ein anderes Salz überführt.

**25.** Eine Verbindung der Formel

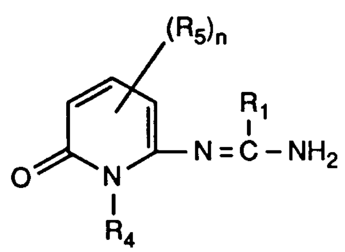

(III')

oder ein Tautomeres und/oder Salz davon, worin $R_1$, $R_4$, $R_5$ und n die in einem der Ansprüche 1-11 angegebenen Bedeutungen haben.

**Patentansprüche für folgende Vertragsstaaten : AT, ES, GR**

**1.** Verfahren zur Herstellung von Verbindungen der Formel

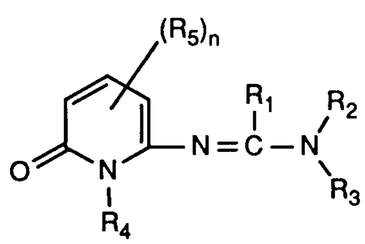

(I)

oder ihrer Tautomeren und/oder ihrer Salze, worin $R_1$ Wasserstoff oder $C_1$-$C_7$-Alkyl bedeutet, einer der Reste $R_2$ und $R_3$ Wasserstoff, $C_1$-$C_7$-Alkyl, Phenyl- oder Naphthyl-$C_1$-$C_7$-alkyl, $C_3$-$C_6$-Alkenyl oder $C_3$-$C_6$-Alkinyl und der andere $C_1$-$C_7$-Alkyl, Phenyl- oder Naphthyl-$C_1$-$C_7$-alkyl, $C_3$-$C_6$-Alkenyl oder $C_3$-$C_6$-Alkinyl bedeutet, $R_4$ Wasserstoff, $C_1$-$C_7$-Alkyl oder Phenyl- oder Naphthyl-$C_1$-$C_7$-alkyl bedeutet, $R_5$ $C_1$-$C_7$-Alkyl oder Trifluormethyl bedeutet und der Index n 0, 1 oder 2 bedeutet, dadurch gekennzeichnet,

dass man

a) eine Verbindung der Formel

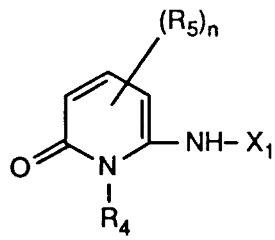

(IIa)

oder ein Tautomeres davon mit einer Verbindung der Formel

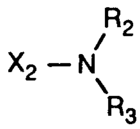

(IIb),

worin einer der Reste $X_1$ und $X_2$ für eine Gruppe der Formel -CO-$R_1$ steht und der andere Wasserstoff oder eine Gruppe der Formel -CO-$Z_1$ ist, worin $Z_1$ einen abspaltbaren Rest bedeutet, oder einem Tautomeren, Salz und/oder Acetal davon umsetzt oder

b) in einer Verbindung der Formel

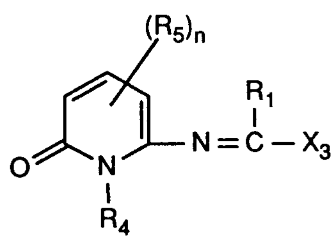

(III)

oder einem Tautomeren und/oder Salz davon, worin $X_3$ einen in

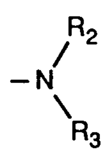

überführbaren Rest bedeutet, $X_3$ in

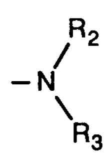

überführt oder

c) eine Verbindung der Formel

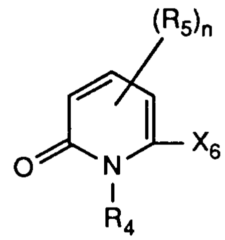

(Va)

oder ein Tautomeres oder Salz davon mit einer Verbindung der Formel

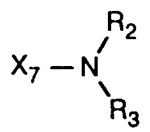

(Vb)

oder einem Tautomeren oder einem Salz davon, wobei $X_6$ für die Gruppe $-N=CR_1-NH-X_6$ steht und $X_7$ für Wasserstoff steht oder $X_6$ für $-NH_2$ steht und $X_7$ für die Gruppe $-CR_1=N-X_6$ steht und $X_6$ für eine Abgangsgruppe steht, umsetzt

und gewünschtenfalls jeweils eine verfahrensgemäss oder auf andere Weise erhältliche Verbindung der Formel I oder ein Tautomeres davon in eine andere Verbindung der Formel I bzw. ein Tautomeres davon überführt, ein verfahrensgemäss erhältliches Isomerengemisch in die Komponenten auftrennt und/oder eine verfahrensgemäss erhältliche freie Verbindung der Formel I bzw. ein Tautomeres davon in ein Salz überführt oder ein verfahrensgemäss erhältliches Salz in die freie Verbindung der Formel I bzw. ein Tautomeres davon oder in ein anderes Salz überführt.

**2.** Verfahren zur Herstellung von Verbindungen der Formel I

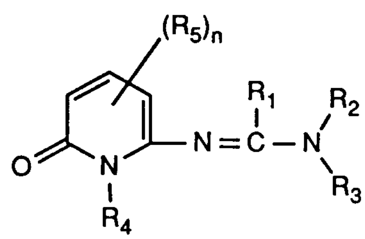

(I)

oder ihrer Tautomeren und/oder ihrer Salze, worin $R_1$ Wasserstoff oder $C_1$-$C_7$-Alkyl bedeutet, einer der Reste $R_2$ und $R_3$ Wasserstoff, $C_1$-$C_7$-Alkyl, Phenyl- oder Naphthyl-$C_1$-$C_7$-alkyl, $C_3$-$C_6$-Alkenyl oder $C_3$-$C_6$-Alkinyl und der andere $C_1$-$C_7$-Alkyl, Phenyl- oder Naphthyl-$C_1$-$C_7$-alkyl, $C_3$-$C_6$-Alkenyl oder $C_3$-$C_6$-Alkinyl bedeutet, $R_4$ Wasserstoff, $C_1$-$C_7$-Alkyl oder Phenyl- oder Naphthyl-$C_1$-$C_7$-alkyl bedeutet, $R_5$ $C_1$-$C_7$-Alkyl oder Trifluormethyl bedeutet und der Index n 0, 1 oder 2 bedeutet, dadurch gekennzeichnet, dass man

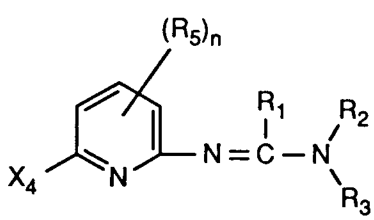

(IV)

oder einem Salz davon, worin $X_4$ für geschütztes Hydroxy steht, die Hydroxyschutzgruppe abspaltet und gewünschtenfalls eine verfahrensgemäss oder auf andere Weise erhältliche Verbindung der Formel I oder ein Tautomeres davon in eine andere Verbindung der Formel I bzw. ein Tautomeres davon überführt, ein verfahrensgemäss erhältliches Isomerengemisch in die Komponenten auftrennt und/oder eine verfahrensgemäss erhältliche freie Verbindung der Formel I bzw. ein Tautomeres davon in ein Salz überführt oder ein verfahrensgemäss erhältliches Salz in die freie Verbindung der Formel I bzw. ein Tautomeres davon oder in ein anderes Salz überführt.

**3.** Verfahren gemäss Anspruch 1 oder 2 zur Herstellung von Verbindungen der Formel I oder ihrer Tautomeren und/oder ihrer Salze, worin $R_1$ Wasserstoff oder $C_1$-$C_7$-Alkyl bedeutet, einer der Reste $R_2$ und $R_3$ Wasserstoff, $C_1$-$C_7$-Alkyl oder Phenyl- oder Naphthyl-$C_1$-$C_7$-alkyl und der andere $C_1$-$C_7$-Alkyl oder Phenyl- oder Naphthyl-$C_1$-$C_7$-alkyl bedeutet, $R_4$ Wasserstoff oder $C_1$-$C_7$-Alkyl bedeutet, $R_5$ $C_1$-$C_7$-Alkyl bedeutet und der Index n 0 oder 1 ist.

**4.** Verfahren gemäss Anspruch 1 oder 2 zur Herstellung von Verbindungen der Formel I oder ihrer Tautomeren und/oder ihrer Salze, worin $R_1$ Wasserstoff oder $C_1$-$C_7$-Alkyl bedeutet, einer der Reste $R_2$ und $R_3$ Wasserstoff, $C_1$-$C_7$-Alkyl oder Phenyl- oder Naphthyl-$C_1$-$C_7$-alkyl und der andere $C_1$-$C_7$-Alkyl oder Phenyl- oder Naphthyl-$C_1$-$C_7$-alkyl bedeutet, $R_4$ Wasserstoff bedeutet und der Index n 0 ist.

**5.** Verfahren gemäss einem der Ansprüche 1 bis 4 zur Herstellung von Verbindungen der Formel I oder ihrer Tautomeren und/oder ihrer Salze, worin einer der Reste $R_2$ und $R_3$ Wasserstoff, $C_1$-$C_7$-Alkyl oder Phenyl- oder Naphthyl-$C_1$-$C_7$-alkyl und der andere $C_1$-$C_7$-Alkyl oder Phenyl- oder Naphthyl-$C_1$-$C_7$-alkyl bedeutet.

**6.** Verfahren gemäss Anspruch 4 zur Herstellung von Verbindungen der Formel I oder ihrer Tautomeren und/oder Salze, worin $R_1$ Wasserstoff oder $C_1$-$C_4$-Alkyl bedeutet, $R_2$ und $R_3$ unabhängig voneinander $C_1$-$C_4$-Alkyl bedeuten oder $R_2$ $C_1$-$C_4$-Alkyl bedeutet und $R_3$ Phenyl-$C_1$-$C_4$-alkyl bedeutet, $R_4$ Wasserstoff ist und der Index n 0 ist.

**7.** Verfahren gemäss Anspruch 1 zur Herstellung von Verbindungen der Formel I oder ihrer Tautomeren und/oder Salze, worin $R_1$ Wasserstoff oder $C_1$-$C_4$-Alkyl bedeutet, $R_2$ und $R_3$ unabhängig voneinander $C_1$-$C_4$-Alkyl bedeuten oder $R_2$ $C_1$-$C_4$-Alkyl bedeutet und $R_3$ Phenyl-$C_1$-$C_4$-alkyl bedeutet, $R_4$ $C_1$-$C_4$-Alkyl ist und der Index n 0 ist.

**8.** Verfahren gemäss Anspruch 4 zur Herstellung von Verbindungen der Formel I oder ihrer Tautomeren und/oder Salze, worin $R_1$ und $R_4$ Wasserstoff bedeuten, $R_2$ und $R_3$ unabhängig voneinander $C_1$-$C_4$-Alkyl bedeuten und der Index n 0 ist.

**9.** Verfahren gemäss Anspruch 1 zur Herstellung von Verbindungen der Formel I oder ihrer Tautomeren und/oder Salze, worin $R_1$ Wasserstoff bedeutet, $R_4$ $C_1$-$C_4$-Alkyl bedeutet, $R_2$ und $R_3$ unabhängig voneinander $C_1$-$C_4$-Alkyl bedeuten und der Index n 0 ist.

**10.** Verfahren gemäss Anspruch 3 zur Herstellung von Verbindungen der Formel I oder ihrer Tautomeren und/oder Salze, worin $R_1$ Methyl ist, $R_4$ Wasserstoff bedeutet, $R_2$ und $R_3$ unabhängig voneinander $C_1$-$C_4$-Alkyl bedeuten und der Index n 0 ist.

**11.** Verfahren gemäss Anspruch 6 zur Herstellung von Verbindungen der Formel I oder ihrer Tautomeren und/oder Salze, worin $R_1$ Wasserstoff oder Methyl bedeutet, $R_2$ Methyl bedeutet, $R_3$ 2-Phenylethyl bedeutet, $R_4$ Wasserstoff ist und der Index n 0 ist.

**12.** Verfahren gemäss Anspruch 7 zur Herstellung von Verbindungen der Formel I oder ihrer Tautomeren und/oder Salze, worin $R_1$ Wasserstoff oder Methyl bedeutet, $R_2$ Methyl bedeutet, $R_3$ 2-Phenylethyl bedeutet, $R_4$ Methyl ist und der Index n 0 ist.

**13.** Verfahren gemäss Anspruch 1 oder 2 zur Herstellung von N-(2-Hydroxy-6-pyridyl)-N',N'-di-n-propyl-formamidin bzw. N-(2-Pyridon-6-yl)-N',N'-di-n-propyl-formamidin oder eines Salzes davon.

**14.** Verfahren gemäss Anspruch 1 oder 2 zur Herstellung von N-(2-Hydroxy-6-pyridyl)-N',N'-di-n-propyl-

acetamidin bzw. N-(2-Pyridon-6-yl)-N',N'-di-n-propyl-acetamidin oder eines Salzes davon.

**15.** Verfahren gemäss Anspruch 1 oder 2 zur Herstellung von N-(2-Hydroxy-6-pyridyl)-N'-methyl-N'-(2-phenylethyl)-acetamidin bzw. N-(2-Pyridon-6-yl)-N'-methyl-N'-(2-phenylethyl)-acetamidin oder eines Salzes davon.

**16.** Verfahren gemäss Anspruch 1 zur Herstellung von N-(1-Methyl-2-pyridon-6-yl)-N',N'-di-n-propylformamidin oder eines Salzes davon.

**17.** Verfahren gemäss Anspruch 1 zur Herstellung von N-(1-Methyl-2-pyridon-6-yl)-N',N'-dipropyl-acetamidin oder eines Salzes davon.

**18.** Verfahren gemäss Anspruch 1 zur Herstellung von N-(1-Benzyl-2-pyridon-6-yl)-N',N'-dipropyl-formamidin oder eines Salzes davon.

**19.** Verfahren zur Herstellung von Verbindungen der Formel

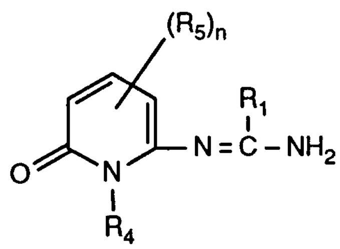

(III')

oder ihrer Tautomeren und/oder Salze, worin $R_1$, $R_4$, $R_5$ und n die in einem der Ansprüche 1-12 angegebenen Bedeutungen haben, dadurch gekennzeichnet, dass man eine Verbindung der Formel

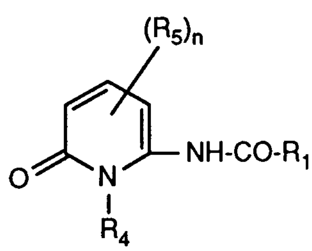

(IIIa)

oder ein Tautomeres und/oder Salz davon mit Ammoniak umsetzt.

**20.** Verfahren zur Herstellung eines pharmazeutischen Präparates, dadurch gekennzeichnet, dass man eine Verbindung, erhältlich gemäss einem der Ansprüche 1 bis 18, oder ein Tautomeres davon, jeweils in freier Form oder in Form eines pharmazeutisch verwendbaren Salzes, gegebenenfalls unter Beimischung von üblichen pharmazeutischen Hilfsstoffen, zu einem pharmazeutischen Präparat verarbeitet.

**21.** Verfahren zur Herstellung eines pharmazeutischen Präparats, dadurch gekennzeichnet, dass man

a) eine Verbindung der Formel

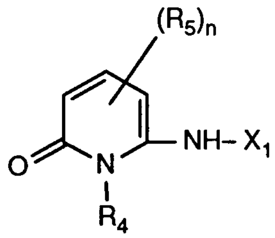

(IIa)

oder ein Tautomeres davon mit einer Verbindung der Formel

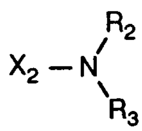

(IIb),

worin einer der Reste $X_1$ und $X_2$ für eine Gruppe der Formel -CO-$R_1$ steht und der andere Wasserstoff oder eine Gruppe der Formel -CO-$Z_1$ ist, worin $Z_1$ einen abspaltbaren Rest bedeutet, oder einem Tautomeren, Salz und/oder Acetal davon umsetzt oder
b) in einer Verbindung der Formel

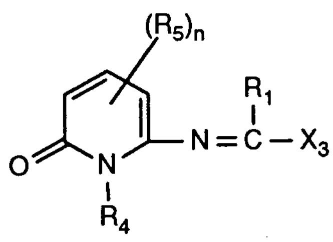

(III)

oder einem Tautomeren und/oder Salz davon, worin $X_3$ einen in

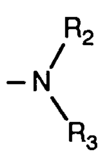

überführbaren Rest bedeutet, $X_3$ in

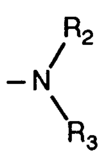

überführt oder
c) eine Verbindung der Formel

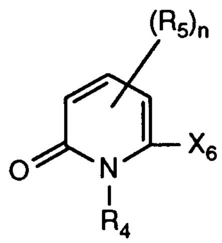

(Va)

oder ein Tautomeres oder Salz davon mit einer Verbindung der Formel

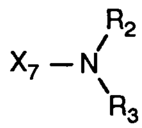

(Vb)

oder einem Tautomeren oder einem Salz davon, wobei $X_6$ für die Gruppe -N = $CR_1$-NH-$X_6$ steht und $X_7$ für Wasserstoff steht oder $X_6$ für -$NH_2$ steht und $X_7$ für die Gruppe -$CR_1$ = N-$X_6$ steht und $X_6$ für eine Abgangsgruppe steht, umsetzt
und gewünschtenfalls jeweils eine verfahrensgemäss oder auf andere Weise erhältliche Verbindung der Formel I oder ein Tautomeres davon in eine andere Verbindung der Formel I bzw. ein Tautomeres davon überführt, ein verfahrensgemäss erhältliches Isomerengemisch in die Komponenten auftrennt und/oder eine verfahrensgemäss erhältliche freie Verbindung der Formel I bzw. ein Tautomeres davon in ein Salz überführt oder ein verfahrensgemäss erhältliches Salz in die freie Verbindung der Formel I bzw. ein Tautomeres davon oder in ein anderes Salz über führt und eine auf diese Weise erhaltene Verbindung der Formel

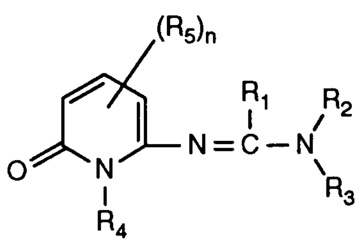

(I),

worin $R_1$ Wasserstoff oder $C_1$-$C_7$-Alkyl bedeutet, einer der Reste $R_2$ und $R_3$ Wasserstoff, $C_1$-$C_7$-Alkyl, Phenyl- oder Naphthyl-$C_1$-$C_7$-alkyl, $C_3$-$C_6$-Alkenyl oder $C_3$-$C_6$-Alkinyl und der andere $C_1$-$C_7$-Alkyl, Phenyl- oder Naphthyl-$C_1$-$C_7$-alkyl, $C_3$-$C_6$-Alkenyl oder $C_3$-$C_6$-Alkinyl bedeutet, $R_4$ Wasserstoff, $C_1$-$C_7$-Alkyl oder Phenyl- oder Naphthyl-$C_1$-$C_7$-alkyl bedeutet, $R_5$ $C_1$-$C_7$-Alkyl oder Trifluormethyl bedeutet und der Index n 0, 1 oder 2 bedeutet, oder ein auf diese Weise erhaltenes Tautomeres davon, jeweils in freier oder in Form eines pharmazeutische verwendbaren Salzes, gegebenenfalls unter Beimischung von üblichen pharmazeutischen Hilfsstoffen, zu einem pharmazeutischen Präparat verarbeitet.

**22.** Verfahren zur Herstellung eines pharmazeutischen Präparats, dadurch gekennzeichnet, dass man in einer Verbindung der Formel

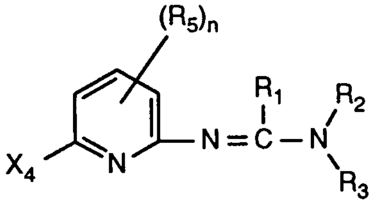

(IV)

oder einem Salz davon, worin $X_4$ für geschütztes Hydroxy steht, die Hydroxyschutzgruppe abspaltet und gewünschtenfalls eine verfahrensgemäss oder auf andere Weise erhältliche Verbindung der Formel I oder ein Tautomeres davon in eine andere Verbindung der Formel I bzw. ein Tautomeres davon überführt, ein verfahrensgemäss erhältliches Isomerengemisch in die Komponenten auftrennt und/oder eine verfahrensgemäss erhältliche freie Verbindung der Formel I bzw. ein Tautomeres davon in ein Salz überführt oder ein verfahrensgemäss erhältliches Salz in die freie Verbindung der Formel I bzw. ein Tautomeres davon oder in ein anderes Salz überführt und eine auf diese Weise erhaltene Verbindung der Formel

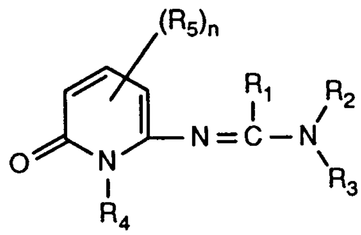

(I),

worin $R_1$ Wasserstoff oder $C_1$-$C_7$-Alkyl bedeutet, einer der Reste $R_2$ und $R_3$ Wasserstoff, $C_1$-$C_7$-Alkyl, Phenyl- oder Naphthyl-$C_1$-$C_7$-alkyl, $C_3$-$C_6$-Alkenyl oder $C_3$-$C_6$-Alkinyl und der andere $C_1$-$C_7$-Alkyl, Phenyl- oder Naphthyl-$C_1$-$C_7$-alkyl, $C_3$-$C_6$-Alkenyl oder $C_3$-$C_6$-Alkinyl bedeutet, $R_4$ Wasserstoff, $C_1$-$C_7$-Alkyl oder Phenyl- oder Naphthyl-$C_1$-$C_7$-alkyl bedeutet, $R_5$ $C_1$-$C_7$-Alkyl oder Trifluormethyl bedeutet und der Index n 0, 1 oder 2 bedeutet, oder ein auf diese Weise erhaltenes Tautomeres davon, jeweils in freier oder in Form eines pharmazeutische verwendbaren Salzes, gegebenenfalls unter Beimischung von üblichen pharmazeutischen Hilfsstoffen, zu einem pharmazeutischen Präparat verarbeitet.

**23.** Verfahren gemäss einem der Ansprüche 20 bis 22 zur Herstellung eines nootropen, antidepressiven und antiparkinsonaktiven pharmazeutischen Präparats, dadurch gekennzeichnet, dass man einen nootropen, antidepressiven und antiparkinsonaktiven Wirkstoff wählt.

**24.** Verwendung einer Verbindung oder eines Tautomeren davon, erhältlich gemäss einem der Ansprüche 1 bis 18, in freier Form oder in Form eines pharmazeutisch verwendbaren Salzes, zur Herstellung eines pharmazeutischen Präparats.

**25.** Verwendung einer Verbindung oder eines Tautomeren davon gemäss Anspruch 24 zur Herstellung eines nootropen, antidepressiven und antiparkinsonaktiven pharmazeutischen Präparats.

**Claims**

**Claims for the following Contracting States : BE, CH, DE, FR, GB, IT, LI, LU, NL, SE**

**1.** A pyridine derivative of the formula

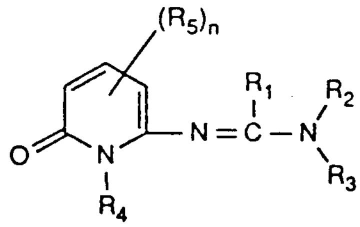

(I),

or a tautomer and/or a salt thereof, in which $R_1$ represents hydrogen or $C_1$-$C_7$-alkyl, one of the radicals $R_2$ and $R_3$ represents hydrogen, $C_1$-$C_7$-alkyl, phenyl- or naphthyl-$C_1$-$C_7$-alkyl, $C_3$-$C_6$-alkenyl or $C_3$-$C_6$-alkynyl and the other represents $C_1$-$C_7$-alkyl, phenyl- or naphthyl-$C_1$-$C_7$-alkyl, $C_3$-$C_6$-alkenyl or $C_3$-$C_6$-alkynyl, $R_4$ represents hydrogen, $C_1$-$C_7$-alkyl or phenyl- or naphthyl-$C_1$-$C_7$-alkyl, $R_5$ represents $C_1$-$C_7$-alkyl or trifluoromethyl, and the index $\underline{n}$ represents 0, 1 or 2.

**2.** A compound according to claim 1 of the formula I or a tautomer and/or a salt thereof, in which $R_1$ represents hydrogen or $C_1$-$C_7$-alkyl, one of the radicals $R_2$ and $R_3$ represents hydrogen, $C_1$-$C_7$-alkyl or phenyl- or naphthyl-$C_1$-$C_7$-alkyl and the other represents $C_1$-$C_7$-alkyl or phenyl- or naphthyl-$C_1$-$C_7$-alkyl, $R_4$ represents hydrogen or $C_1$-$C_7$-alkyl, $R_5$ represents $C_1$-$C_7$-alkyl, and the index $\underline{n}$ represents 0 or 1.

**3.** A compound according to claim 1 of the formula I or a tautomer and/or a salt thereof, in which $R_1$ represents hydrogen or $C_1$-$C_7$-alkyl, one of the radicals $R_2$ and $R_3$ represents hydrogen, $C_1$-$C_7$-alkyl or phenyl- or naphthyl-$C_1$-$C_7$-alkyl and the other represents $C_1$-$C_7$-alkyl or phenyl- or naphthyl-$C_1$-$C_7$-alkyl, $R_4$ represents hydrogen, and the index $\underline{n}$ represents 0.

**4.** A compound according to claim 1, 2 or 3 of the formula I or a tautomer and/or a salt thereof, in which one of the radicals $R_2$ and $R_3$ represents hydrogen, $C_1$-$C_7$-alkyl or phenyl- or naphthyl-$C_1$-$C_7$-alkyl and the other represents $C_1$-$C_7$-alkyl or phenyl- or naphthyl-$C_1$-$C_7$-alkyl.

**5.** A compound according to claim 3 of the formula I or a tautomer and/or a salt thereof, in which $R_1$ represents hydrogen or $C_1$-$C_4$-alkyl, each of $R_2$ and $R_3$, independently of the other, represents $C_1$-$C_4$-alkyl, or $R_2$ represents $C_1$-$C_4$-alkyl and $R_3$ represents phenyl-$C_1$-$C_4$-alkyl, $R_4$ represents hydrogen, and the index $\underline{n}$ represents 0.

**6.** A compound according to claim 2 of the formula I or a tautomer and/or a salt thereof, in which $R_1$ represents hydrogen or $C_1$-$C_4$-alkyl, each of $R_2$ and $R_3$, independently of the other, represents $C_1$-$C_4$-alkyl, or $R_2$ represents $C_1$-$C_4$-alkyl and $R_3$ represents phenyl-$C_1$-$C_4$-alkyl, $R_4$ represents $C_1$-$C_4$-alkyl, and the index $\underline{n}$ represents 0.

**7.** A compound according to claim 3 of the formula I or a tautomer and/or a salt thereof, in which $R_1$ and $R_4$ represent hydrogen, each of $R_2$ and $R_3$, independently of the other, represents $C_1$-$C_4$-alkyl, and the index $\underline{n}$ represents 0.

**8.** A compound according to claim 2 of the formula I or a tautomer and/or a salt thereof, in which $R_1$ represents hydrogen, $R_4$ represents $C_1$-$C_4$-alkyl, each of $R_2$ and $R_3$, independently of the other, represents $C_1$-$C_4$-alkyl, and the index $\underline{n}$ represents 0.

**9.** A compound according to claim 2 of the formula I or a tautomer and/or a salt thereof, in which $R_1$ represents methyl, $R_4$ represents hydrogen, each of $R_2$ and $R_3$, independently of the other, represents $C_1$-$C_4$-alkyl, and the index $\underline{n}$ represents 0.

**10.** A compound according to claim 5 of the formula I or a tautomer and/or a salt thereof, in which $R_1$ represents hydrogen or methyl, $R_2$ represents methyl, $R_3$ represents 2-phenylethyl, $R_4$ represents hydrogen, and the index $\underline{n}$ represents 0.

**11.** A compound according to claim 6 of the formula I or a tautomer and/or a salt thereof, in which $R_1$ represents hydrogen or methyl, $R_2$ represents methyl, $R_3$ represents 2-phenylethyl, $R_4$ represents

hydrogen, and the index n represents 0.

**12.** N-(2-hydroxy-6-pyridyl)-N',N'-di-n-propylformamidine or N-(2-pyridon-6-yl)-N',N'-di-n-propylformamidine or a salt thereof.

**13.** N-(2-hydroxy-6-pyridyl)-N',N'-di-n-propylacetamidine or N-(2-pyridon-6-yl)-N',N'-di-n-propylacetamidine or a salt thereof.

**14.** N-(2-hydroxy-6-pyridyl)-N'-methyl-N'-(2-phenylethyl)-acetamidine or N-(2-pyridon-6-yl)-N'-methyl-N'-(2-phenylethyl)-acetamidine or a salt thereof.

**15.** N-(1-methyl-2-pyridon-6-yl)-N',N'-di-n-propylformamidine or a salt thereof.

**16.** N-(1-methyl-2-pyridon-6-yl)-N',N'-dipropylacetamidine or a salt thereof.

**17.** N-(1-benzyl-2-pyridon-6-yl)-N',N'-dipropylformamidine or a salt thereof.

**18.** A compound according to any one of claims 1 to 17 or a tautomer and/or a pharmaceutically acceptable salt thereof for use in a method for the therapeutic or prophylactic treatment of the human or animal body.

**19.** A compound according to any one of claims 1 to 18 or a tautomer and/or a pharmaceutically acceptable salt thereof for use as a nootropic, an antidepressant or an anti-Parkinson agent.

**20.** A pharmaceutical composition comprising a compound or a tautomer according to any one of claims 1 to 19 in free form or in the form of a pharmaceutically acceptable salt.

**21.** A pharmaceutical composition according to claim 20 having nootropic, antidepressive and anti-Parkinson action.

**22.** The use of a compound according to any one of claims 1 to 19 or a tautomer and/or a pharmaceutically acceptable salt thereof for the preparation of nootropics, antidepressants and anti-Parkinson agents.

**23.** A process for the preparation of a compound or a tautomer and/or a salt according to any one of claims 1 to 17, wherein

a) a compound of the formula

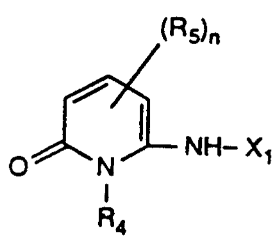

(IIa)

or a tautomer thereof is reacted with a compound of the formula

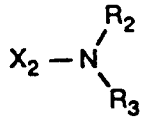

(IIb)

in which one of the radicals $X_1$ and $X_2$ represents a group of the formula -CO-$R_1$ and the other represents hydrogen or a group of the formula -CO-$Z_1$ in which $Z_1$ represents a removable radical,

or a tautomer, salt and/or acetal thereof, or
b) in a compound of the formula

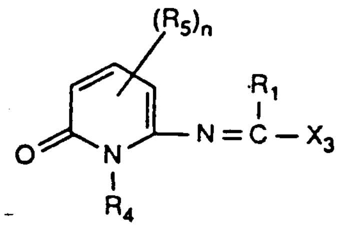

(III)

or a tautomer and/or a salt thereof in which $X_3$ represents a radical that can be converted into

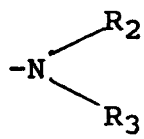

,

$X_3$ is converted into

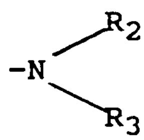

,

or
c) a compound of the formula

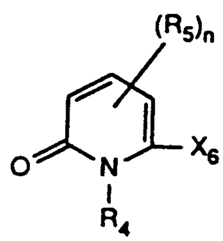

(Va)

or a tautomer or a salt thereof is reacted with a compound of the formula

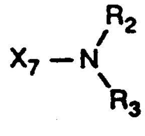

(Vb)

or a tautomer or a salt thereof, in which $X_6$ represents the group $-N=CR_1-NH-X_6$ and $X_7$ represents hydrogen, or $X_6$ represents $-NH_2$ and $X_7$ represents the group $-CR_1=N-X_6$, and $X_6$ represents a leaving group,

and, if desired, a compound of the formula I obtainable according to the process or by other means or a tautomer thereof is converted into a different compound of the formula I or a tautomer thereof, an isomeric mixture obtainable according to the process is separated into its components, and/or a free compound of the formula I obtainable according to the process or a tautomer thereof is converted into a salt, or a salt obtainable according to the process is converted into the free compound of the formula I or a tautomer thereof or into a different salt.

**24.** A process for the preparation of a compound or a tautomer and/or a salt according to any one of claims 1 to 5, 7, 9, 10 and 12 to 14 of the formula I in which $R_4$ represents hydrogen, wherein, in a compound of the formula

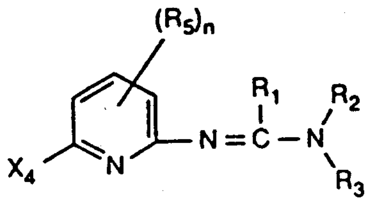

(IV)

or a salt thereof, in which $X_4$ represents protected hydroxy, the hydroxy-protecting group is removed and, if desired, a compound of the formula I obtainable according to the process or by other means or a tautomer thereof is converted into a different compound of the formula I or a tautomer thereof, an isomeric mixture obtainable according to the process is separated into its components, and/or a free compound of the formula I obtainable according to the process or a tautomer thereof is converted into a salt, or a salt obtainable according to the process is converted into the free compound of the formula I or a tautomer thereof or into a different salt.

**25.** A compound of the formula

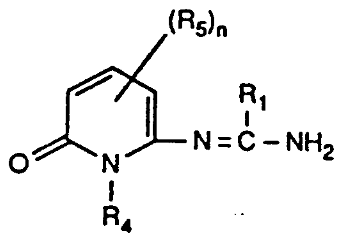

(III')

or a tautomer and/or a salt thereof, in which $R_1$, $R_4$, $R_5$ and $\underline{n}$ have the meanings given in any one of claims 1 to 11.

**Claims for the following Contracting States : AT, ES, GR**

**1.** A process for the preparation of a compound of the formula

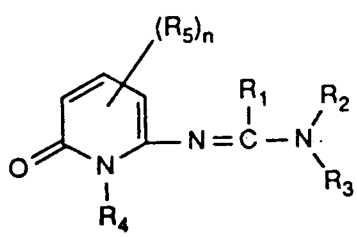

(I),

or a tautomer and/or a salt thereof, in which $R_1$ represents hydrogen or $C_1$-$C_7$-alkyl, one of the radicals $R_2$ and $R_3$ represents hydrogen, $C_1$-$C_7$-alkyl, phenyl- or naphthyl-$C_1$-$C_7$-alkyl, $C_3$-$C_6$-alkenyl or $C_3$-$C_6$-alkynyl and the other represents $C_1$-$C_7$-alkyl, phenyl- or naphthyl-$C_1$-$C_7$-alkyl, $C_3$-$C_6$-alkenyl or $C_3$-$C_6$-alkynyl, $R_4$ represents hydrogen, $C_1$-$C_7$-alkyl or phenyl- or naphthyl-$C_1$-$C_7$-alkyl, $R_5$ represents $C_1$-$C_7$-alkyl or trifluoromethyl, and the index $\underline{n}$ represents 0, 1 or 2, wherein

a) a compound of the formula

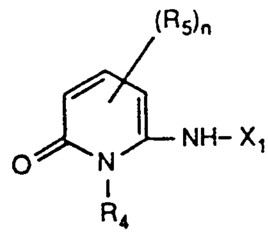

(IIa)

or a tautomer thereof is reacted with a compound of the formula

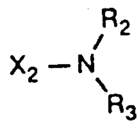

(IIb)

in which one of the radicals $X_1$ and $X_2$ represents a group of the formula -CO-$R_1$ and the other represents hydrogen or a group of the formula -CO-$Z_1$ in which $Z_1$ represents a removable radical, or a tautomer, salt and/or acetal thereof, or

b) in a compound of the formula

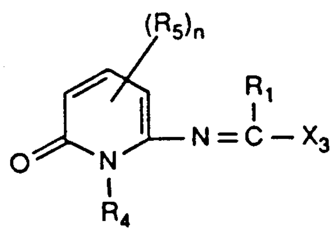

(III)

or a tautomer and/or a salt thereof in which $X_3$ represents a radical that can be converted into

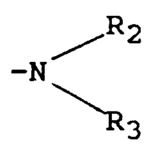

,

$X_3$ is converted into

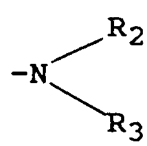

,

or

c) a compound of the formula

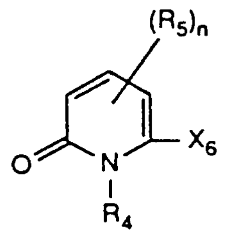

(Va)

or a tautomer or a salt thereof is reacted with a compound of the formula

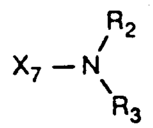

(Vb)

or a tautomer or a salt thereof, in which $X_6$ represents the group $-N=CR_1-NH-X_6$ and $X_7$ represents hydrogen, or $X_6$ represents $-NH_2$ and $X_7$ represents the group $-CR_1=N-X_6$, and $X_6$ represents a leaving group,

and, if desired, a compound of the formula I obtainable according to the process or by other means or a tautomer thereof is converted into a different compound of the formula I or a tautomer thereof, an isomeric mixture obtainable according to the process is separated into its components, and/or a free compound of the formula I obtainable according to the process or a tautomer thereof is converted into a salt, or a salt obtainable according to the process is converted into the free compound of the formula I or a tautomer thereof or into a different salt.

**2.** A process for the preparation of a compound of the formula I

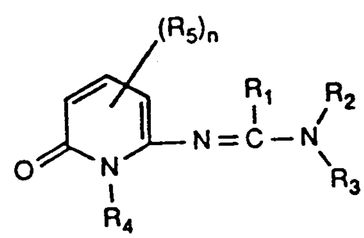

(I)

or a tautomer and/or a salt thereof, in which $R_1$ represents hydrogen or $C_1$-$C_7$-alkyl, one of the radicals $R_2$ and $R_3$ represents hydrogen, $C_1$-$C_7$-alkyl, phenyl- or naphthyl-$C_1$-$C_7$-alkyl, $C_3$-$C_6$-alkenyl or $C_3$-$C_6$-alkynyl and the other represents $C_1$-$C_7$-alkyl, phenyl- or naphthyl-$C_1$-$C_7$-alkyl, $C_3$-$C_6$-alkenyl or $C_3$-$C_6$-alkynyl, $R_4$ represents hydrogen, $C_1$-$C_7$-alkyl or phenyl- or naphthyl-$C_1$-$C_7$-alkyl, $R_5$ represents $C_1$-$C_7$-alkyl or trifluoromethyl, and the index n represents 0, 1 or 2, wherein

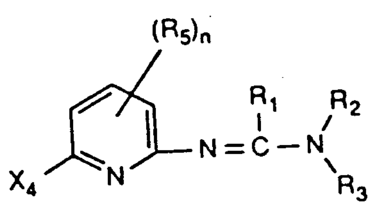

(IV)

or a salt thereof, in which $X_4$ represents protected hydroxy, the hydroxy-protecting group is removed and, if desired, a compound of the formula I obtainable according to the process or by other means or a tautomer thereof is converted into a different compound of the formula I or a tautomer thereof, an isomeric mixture obtainable according to the process is separated into its components, and/or a free

compound of the formula I obtainable according to the process or a tautomer thereof is converted into a salt, or a salt obtainable according to the process is converted into the free compound of the formula I or a tautomer thereof or into a different salt.

**3.** A process according to claim 1 or 2 for the preparation of a compound of the formula I or a tautomer and/or a salt thereof, in which $R_1$ represents hydrogen or $C_1$-$C_7$-alkyl, one of the radicals $R_2$ and $R_3$ represents hydrogen, $C_1$-$C_7$-alkyl or phenyl- or naphthyl-$C_1$-$C_7$-alkyl and the other represents $C_1$-$C_7$-alkyl or phenyl- or naphthyl-$C_1$-$C_7$-alkyl, $R_4$ represents hydrogen or $C_1$-$C_7$-alkyl, $R_5$ represents $C_1$-$C_7$-alkyl, and the index $\underline{n}$ represents 0 or 1.

**4.** A process according to claim 1 or 2 for the preparation of a compound of the formula I or a tautomer and/or a salt thereof, in which $R_1$ represents hydrogen or $C_1$-$C_7$-alkyl, one of the radicals $R_2$ and $R_3$ represents hydrogen, $C_1$-$C_7$-alkyl or phenyl- or naphthyl-$C_1$-$C_7$-alkyl and the other represents $C_1$-$C_7$-alkyl or phenyl- or naphthyl-$C_1$-$C_7$-alkyl, $R_4$ represents hydrogen, and the index $\underline{n}$ represents 0.

**5.** A process according to any one of claims 1 to 4 for the preparation of a compound of the formula I or a tautomer and/or a salt thereof, in which one of the radicals $R_2$ and $R_3$ represents hydrogen, $C_1$-$C_7$-alkyl or phenyl- or naphthyl-$C_1$-$C_7$-alkyl and the other represents $C_1$-$C_7$-alkyl or phenyl- or naphthyl-$C_1$-$C_7$-alkyl.

**6.** A process according to claim 4 for the preparation of a compound of the formula I or a tautomer and/or a salt thereof, in which $R_1$ represents hydrogen or $C_1$-$C_4$-alkyl, each of $R_2$ and $R_3$, independently of the other, represents $C_1$-$C_4$-alkyl, or $R_2$ represents $C_1$-$C_4$-alkyl and $R_3$ represents phenyl-$C_1$-$C_4$-alkyl, $R_4$ represents hydrogen, and the index $\underline{n}$ represents 0.

**7.** A process according to claim 1 for the preparation of a compound of the formula I or a tautomer and/or a salt thereof, in which $R_1$ represents hydrogen or $C_1$-$C_4$-alkyl, each of $R_2$ and $R_3$, independently of the other, represents $C_1$-$C_4$-alkyl, or $R_2$ represents $C_1$-$C_4$-alkyl and $R_3$ represents phenyl-$C_1$-$C_4$-alkyl, $R_4$ represents $C_1$-$C_4$-alkyl, and the index $\underline{n}$ represents 0.

**8.** A process according to claim 4 for the preparation of a compound of the formula I or a tautomer and/or a salt thereof, in which $R_1$ and $R_4$ represent hydrogen, each of $R_2$ and $R_3$, independently of the other, represents $C_1$-$C_4$-alkyl, and the index $\underline{n}$ represents 0.

**9.** A process according to claim 1 for the preparation of a compound of the formula I or a tautomer and/or a salt thereof, in which $R_1$ represents hydrogen, $R_4$ represents $C_1$-$C_4$-alkyl, each of $R_2$ and $R_3$, independently of the other, represents $C_1$-$C_4$-alkyl, and the index $\underline{n}$ represents 0.

**10.** A process according to claim 3 for the preparation of a compound of the formula I or a tautomer and/or a salt thereof, in which $R_1$ represents methyl, $R_4$ represents hydrogen, each of $R_2$ and $R_3$, independently of the other, represents $C_1$-$C_4$-alkyl, and the index $\underline{n}$ represents 0.

**11.** A process according to claim 6 for the preparation of a compound of the formula I or a tautomer and/or a salt thereof, in which $R_1$ represents hydrogen or methyl, $R_2$ represents methyl, $R_3$ represents 2-phenylethyl, $R_4$ represents hydrogen, and the index $\underline{n}$ represents 0.

**12.** A process according to claim 7 for the preparation of a compound of the formula I or a tautomer and/or a salt thereof, in which $R_1$ represents hydrogen or methyl, $R_2$ represents methyl, $R_3$ represents 2-phenylethyl, $R_4$ represents methyl, and the index $\underline{n}$ represents 0.

**13.** A process according to claim 1 or 2 for the preparation of N-(2-hydroxy-6-pyridyl)-N',N'-di-n-propylformamidine or N-(2-pyridon-6-yl)-N',N'-di-n-propylformamidine or a salt thereof.

**14.** A process according to claim 1 or 2 for the preparation of N-(2-hydroxy-6-pyridyl)-N',N'-di-n-propylacetamidine or N-(2-pyridon-6-yl)-N',N'-di-n-propylacetamidine or a salt thereof.

**15.** A process according to claim 1 or 2 for the preparation of N-(2-hydroxy-6-pyridyl)-N'-methyl-N'-(2-phenylethyl)-acetamidine or N-(2-pyridon-6-yl)-N'-methyl-N'-(2-phenylethyl)-acetamidine or a salt thereof.

**16.** A process according to claim 1 for the preparation of N-(1-methyl-2-pyridon-6-yl)-N',N'-di-n-propylformamidine or a salt thereof.

**17.** A process according to claim 1 for the preparation of N-(1-methyl-2-pyridon-6-yl)-N',N'-dipropylacetamidine or a salt thereof.

**18.** A process according to claim 1 for the preparation of N-(1-benzyl-2-pyridon-6-yl)-N',N'-dipropylformamidine or a salt thereof.

**19.** A process for the preparation of a compound of the formula

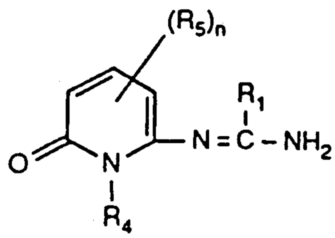

(III')

or a tautomer and/or a salt thereof, in which $R_1$, $R_4$, $R_5$ and n have the meanings given in any one of claims 1 to 12, wherein a compound of the formula

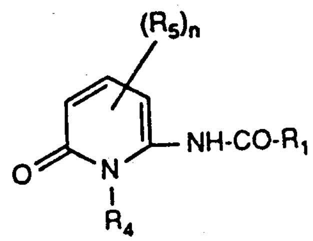

(IIIa)

or a tautomer and/or a salt thereof is reacted with ammonia.

**20.** A process for the preparation of a pharmaceutical composition, wherein a compound, obtainable according to any one of claims 1 to 18, or a tautomer thereof, in each case in free form or in the form of a pharmaceutically acceptable salt, is processed to form a pharmaceutical composition, optionally with the admixture of customary pharmaceutical excipients.

**21.** A process for the preparation of a pharmaceutical composition, wherein
a) a compound of the formula

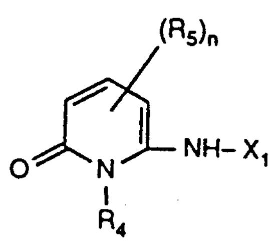

(IIa)

or a tautomer thereof is reacted with a compound of the formula

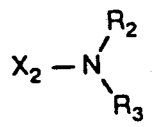

(IIb)

in which one of the radicals $X_1$ and $X_2$ represents a group of the formula -CO-$R_1$ and the other represents hydrogen or a group of the formula -CO-$Z_1$ in which $Z_1$ represents a removable radical, or a tautomer, salt and/or acetal thereof, or

b) in a compound of the formula

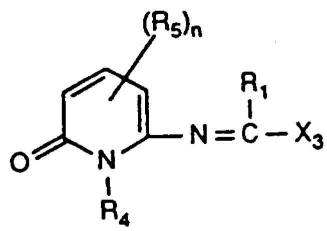

(III)

or a tautomer and/or a salt thereof in which $X_3$ represents a radical that can be converted into

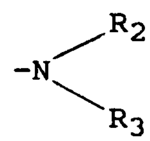

,

$X_3$ is converted into

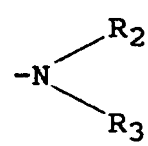

,

or

c) a compound of the formula

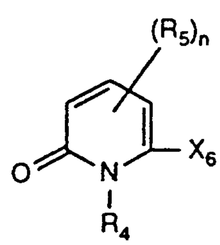

(Va)

or a tautomer or a salt thereof is reacted with a compound of the formula

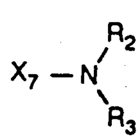

(Vb)

or a tautomer or a salt thereof, in which $X_6$ represents the group $-N=CR_1-NH-X_6$ and $X_7$ represents hydrogen, or $X_6$ represents $-NH_2$ and $X_7$ represents the group $-CR_1=N-X_6$, and $X_6$ represents a leaving group,

and, if desired, a compound of the formula I obtainable according to the process or by other means or a tautomer thereof is converted into a different compound of the formula I or a tautomer thereof, an isomeric mixture obtainable according to the process is separated into its components, and/or a free compound of the formula I obtainable according to the process or a tautomer thereof is converted into a salt, or a salt obtainable according to the process is converted into the free compound of the formula I or a tautomer thereof or into a different salt, and a compound of the formula

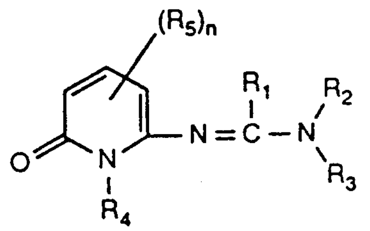

(I)

obtained in that manner in which $R_1$ represents hydrogen or $C_1$-$C_7$-alkyl, one of the radicals $R_2$ and $R_3$ represents hydrogen, $C_1$-$C_7$-alkyl, phenyl- or naphthyl-$C_1$-$C_7$-alkyl, $C_3$-$C_6$-alkenyl or $C_3$-$C_6$-alkynyl and the other represents $C_1$-$C_7$-alkyl, phenyl- or naphthyl-$C_1$-$C_7$-alkyl, $C_3$-$C_6$-alkenyl or $C_3$-$C_6$-alkynyl, $R_4$ represents hydrogen, $C_1$-$C_7$-alkyl or phenyl- or naphthyl-$C_1$-$C_7$-alkyl, $R_5$ represents $C_1$-$C_7$-alkyl or trifluoromethyl, and the index n represents 0, 1 or 2, or a tautomer obtained in that manner, in each case in free form or in the form of a pharmaceutically acceptable salt, is processed to form a pharmaceutical composition, optionally with the admixture of customary pharmaceutical excipients.

**22.** A process for the preparation of a pharmaceutical composition, wherein, in a compound of the formula

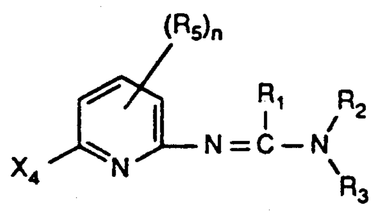

(IV)

or a salt thereof, in which $X_4$ represents protected hydroxy, the hydroxy-protecting group is removed and, if desired, a compound of the formula I obtainable according to the process or by other means or a tautomer thereof is converted into a different compound of the formula I or a tautomer thereof, an isomeric mixture obtainable according to the process is separated into its components, and/or a free compound of the formula I obtainable according to the process or a tautomer thereof is converted into a salt, or a salt obtainable according to the process is converted into the free compound of the formula I or a tautomer thereof or into a different salt, and a compound of the formula

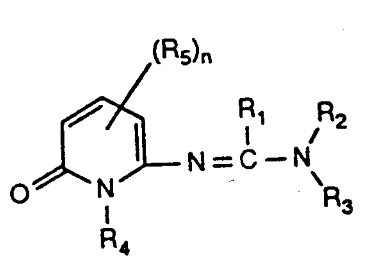

(I)

obtained in that manner in which $R_1$ represents hydrogen or $C_1$-$C_7$-alkyl, one of the radicals $R_2$ and $R_3$ represents hydrogen, $C_1$-$C_7$-alkyl, phenyl- or naphthyl-$C_1$-$C_7$-alkyl, $C_3$-$C_6$-alkenyl or $C_3$-$C_6$-alkynyl and the other represents $C_1$-$C_7$-alkyl, phenyl- or naphthyl-$C_1$-$C_7$-alkyl, $C_3$-$C_6$-alkenyl or $C_3$-$C_6$-alkynyl, $R_4$

represents hydrogen, $C_1$-$C_7$-alkyl or phenyl- or naphthyl-$C_1$-$C_7$-alkyl, $R_5$ represents $C_1$-$C_7$-alkyl or trifluoromethyl, and the index n represents 0, 1 or 2, or a tautomer thereof obtained in that manner, in each case in free form or in the form of a pharmaceutically acceptable salt, is processed to form a pharmaceutical composition, optionally with the admixture of customary pharmaceutical excipients.

**23.** A process according to any one of claims 20 to 22 for the preparation of a pharmaceutical composition having nootropic, antidepressive and anti-Parkinson action, wherein an active ingredient having nootropic, antidepressive and anti-Parkinson action is selected.

**24.** The use of a compound or a tautomer thereof, obtainable according to any one of claims 1 to 18, in free form or in the form of a pharmaceutically acceptable salt, for the preparation of a pharmaceutical composition.

**25.** Use of a compound or a tautomer thereof according to claim 24 for the preparation of a pharmaceutical composition having nootropic, antidepressive and anti-Parkinson action.

**Revendications**
**Revendications pour les Etats contractants suivants : BE, CH, DE, FR, GB, IT, LI, LU, NL, SE**

**1.** Dérivés de la pyridine de formule

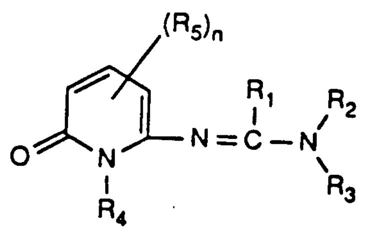

(I)

leurs tautomères et/ou leurs sels où $R_1$ représente l'hydrogène ou un alkyle en $C_1$-$C_7$, l'un des restes $R_2$ et $R_3$ représente l'hydrogène, un alkyle en $C_1$-$C_7$, un phénylalkyle en $C_1$-$C_7$ ou un naphtylalkyle en $C_1$-$C_7$, un un alcényle en $C_3$-$C_6$ ou un alcynyle en $C_3$-$C_6$ et l'autre reste un alkyle en $C_1$-$C_7$, un phénylalkyle en $C_1$-$C_7$ ou un naphtylalkyle en $C_1$-$C_7$, un alcényle en $C_3$-$C_6$ ou un alcynyle en $C_3$-$C_6$, $R_4$ l'hydrogène, un alkyle en $C_1$-$C_7$ ou un phénylalkyle en $C_1$-$C_7$ ou un naphtylalkyle en $C_1$-$C_7$, $R_5$ un alkyle en $C_1$-$C_7$ ou le trifluorométhyle et l'indice n est égal à 0, 1 ou 2.

**2.** Composés selon la revendication 1 de formule I, leurs tautomères et/ou leurs sels, où $R_1$ représente l'hydrogène ou un alkyle en $C_1$-$C_7$, l'un des restes $R_2$ et $R_3$ représente l'hydrogène, un alkyle en $C_1$-$C_7$ ou un phényl- ou naphtylalkyle en $C_1$-$C_7$ et l'autre reste représente un alkyle en $C_1$-$C_7$ ou un phényl- ou naphtylalkyle en $C_1$-$C_7$, $R_4$ représente l'hydrogène ou un alkyle en $C_1$-$C_7$, $R_5$ représente un alkyle en $C_1$-$C_7$ et l'indice n est 0 ou 1.

**3.** Composés selon la revendication 1 de formule I, leurs tautomères et/ou leurs sels, où $R_1$ représente l'hydrogène ou un alkyle en $C_1$-$C_7$, l'un des restes $R_2$ et $R_3$ représente l'hydrogène, un alkyle en $C_1$-$C_7$ ou un phényl- ou naphtylalkyle en $C_1$-$C_7$ et l'autre reste représente un alkyle en $C_1$-$C_7$ ou un phényl- ou naphtylalkyle en $C_1$-$C_7$, $R_4$ représente l'hydrogène et l'indice n est 0.

**4.** Composés selon les revendications 1, 2 ou 3 de formule I, leurs tautomères et/ou leurs sels, où l'un des restes $R_2$ et $R_3$ représente l'hydrogène, un alkyle en $C_1$-$C_7$ ou un phényl- ou naphtylalkyle en $C_1$-$C_7$ et l'autre reste représente un alkyle en $C_1$-$C_7$ ou un phényl- ou naphtylalkyle en $C_1$-$C_7$.

**5.** Composés selon la revendication 3 de formule I, leurs tautomères et/ou leurs sels, où $R_1$ représente l'hydrogène ou un alkyle en $C_1$-$C_4$, $R_2$ et $R_3$ représentent, indépendamment l'un de l'autre, un alkyle en $C_1$-$C_4$ ou $R_2$ représente un alkyle en $C_1$-$C_4$ et $R_3$ représente un phénylalkyle en $C_1$-$C_4$, $R_4$ est l'hydrogène et l'indice n est 0.

**6.** Composés selon la revendication 2 de formule I, leurs tautomères et/ou leurs sels, où $R_1$ représente l'hydrogène ou un alkyle en $C_1$-$C_4$, $R_2$ et $R_3$ représentent, indépendamment l'un de l'autre, un alkyle en $C_1$-$C_4$ ou $R_2$ représente un alkyle en $C_1$-$C_4$ et $R_3$ représente un phénylalkyle en $C_1$-$C_4$, $R_4$ est un alkyle en $C_1$-$C_4$ et l'indice n est égal à 0.

**7.** Composés selon la revendication 3 de formule I, leurs tautomères et/ou leurs sels, où $R_1$ et $R_4$ représentent l'hydrogène, $R_2$ et $R_3$ représentent, indépendamment l'un de l'autre, un alkyle en $C_1$-$C_4$ et l'indice n est 0.

**8.** Composés selon la revendication 2 de formule I, leurs tautomères et/ou leurs sels, où $R_1$ représente l'hydrogène, $R_4$ un alkyle en $C_1$-$C_4$, $R_2$ et $R_3$ représentent, indépendamment l'un de l'autre, un alkyle en $C_1$-$C_4$ et l'indice n est 0.

**9.** Composés selon la revendication 2 de formule I, leurs tautomères et/ou leurs sels, où $R_1$ est le méthyle, $R_4$ représente l'hydrogène, $R_2$ et $R_3$ représentent, indépendamment l'un de l'autre, un alkyle en $C_1$-$C_4$ et l'indice n est 0.

**10.** Composés selon la revendication 5 de formule I, leurs tautomères et/ou leurs sels, où $R_1$ représente l'hydrogène ou le méthyle, $R_2$ le méthyle, $R_3$ le 2-phényléthyle, $R_4$ l'hydrogène et l'indice n est 0.

**11.** Composés selon la revendication 6 de formule I, leurs tautomères et/ou leurs sels, où $R_1$ représente l'hydrogène ou le méthyle, $R_2$ le méthyle, $R_3$ le 2-phényléthyle, $R_4$ est l'hydrogène et l'indice n est 0.

**12.** La N-(2-hydroxy-6-pyridyl)-N',N'-di-n-propylformamidine ou la N-(2-pyridon-6-yl)-N',N'-di-n-propylformamidine ou l'un de leurs sels.

**13.** La N-(2-hydroxy-6-pyridyl)-N',N'-di-n-propylacétamidine ou la N-(2-pyridon-6-yl)-N',N'-di-n-propylacétamidine ou l'un de leurs sels.

**14.** La N-(2-hydroxy-6-pyridyl)-N'-méthyl-N'-(2-phényléthyl)-acétamide ou la N-(2-pyridon-6-yl)-N'-méthyl-N'-(2-phényléthyl)-acétamidine ou l'un de leurs sels.

**15.** La N-(1-méthyl-2-pyridon-6-yl)-N',N'-di-n-propylformamidine ou l'un de ses sels.

**16.** La N-(1-méthyl-2-pyridon-6-yl)-N',N'-di-propyl-acétamidine ou l'un de ses sels.

**17.** La N-(1-benzyl-2-pyridon-6-yl)-N',N'-di-propyl-formamidine ou l'un de ses sels.

**18.** Composé selon l'une des revendications 1 à 17 ou l'un de ses tautomères et/ou l'un de ses sels pharmaceutiquement utilisable destiné à être utilisé dans un procédé pour le traitement thérapeutique ou prophylactique du corps humain ou animal.

**19.** Composé selon l'une des revendications 1 à 18 ou l'un de ses tautomères et/ou l'un de ses sels pharmaceutiquement utilisable destiné à être utilisé comme nootrope, antidépresseur et antiparkinsonien.

**20.** Préparations pharmaceutiques contenant un composé ou un tautomère selon l'une des revendications 1 à 19 sous forme libre ou sous forme d'un sel pharmaceutiquement utilisable.

**21.** Préparation pharmaceutique selon la revendication 20 agissant comme nootrope, antidépresseur et antiparkinsonien.

**22.** utilisation d'un composé selon l'une des revendications 1 à 19 ou de l'un de ses tautomères et/ou de l'un de ses sels pharmaceutiquement utilisable pour la préparation de nootropes, antidépresseurs et antiparkinsoniens.

**23.** Procédé pour la préparation des composés, des tautomères et/ou des sels selon l'une des revendications 1 à 17, caractérisé

a) en ce que l'on fait réagir un composé de formule

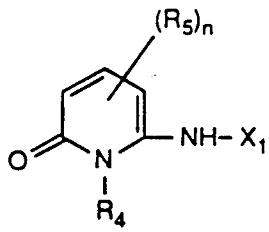

(IIa)

ou l'un de ses tautomères avec un composé de formule

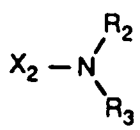

(IIb)

où l'un des restes $X_1$ et $X_2$ représente un groupe de formule $-CO-R_1$ et l'autre reste représente l'hydrogène ou un groupe de formule $-CO-Z_1$, $Z_1$ représentant un reste clivable ou avec l'un de ses tautomères, avec l'un de ses sels et/ou avec l'un de ses acétals ou

b) en ce que l'on transforme dans un composé de formule

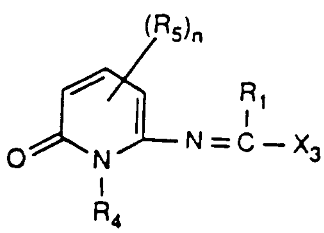

(III)

ou dans l'un de ses tautomères et/ou dans l'un de ses sels, où $X_3$ représente un reste transformable en

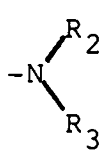

,

$X_3$ en

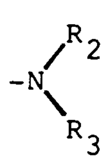

ou

c) en ce que l'on fait réagir un composé de formule

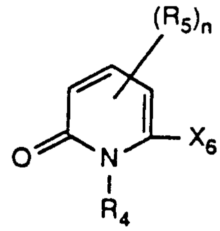

(Va)

ou l'un de ses tautomères ou l'un de ses sels avec un composé

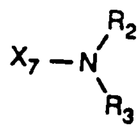

(Vb)

ou avec l'un de ses tautomères ou avec l'un de ses sels, $X_6$ représentant le groupe $-N=CR_1-NH-X_6$ et $X_7$ représentant l'hydrogène ou $X_6$ représentant $-NH_2$ et $X_7$ représentant le groupe $-CR_1=N-X_6$ et $X_6$ représentant un groupe partant, et, si désiré, en ce que l'on transforme un composé de formule I ou l'un de ses tautomères pouvant être obtenu par le procédé conforme à l'invention ou d'une autre façon en un autre composé de formule I ou en l'un de ses tautomères, en ce que l'on sépare le mélange d'isomères pouvant être obtenu par le procédé conforme à l'invention en ses composants et/ou en ce que l'on transforme le composé libre de formule I ou l'un de ses tautomères pouvant être obtenu par le procédé conforme à l'invention en un sel ou le sel pouvant être obtenu par le procédé conforme à l'invention en un composé libre de formule I ou en l'un de ses tautomères ou en un autre sel.

**24.** Procédé pour la préparation des composés, des tautomères et/ou des sels selon l'une des revendications 1 à 5, 7, 9, 10 et 12 à 14 de formule I, où $R_4$ représente l'hydrogène, caractérisé en ce que, dans un composé de formule

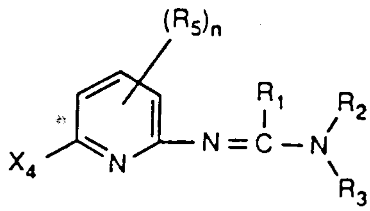

(IV)

ou dans l'un de ses sels, où $X_4$ représente un hydroxy protégé, l'on clive le groupe protecteur de l'hydroxy et, si désiré, en ce que l'on transforme un composé de formule I ou l'un de ses tautomères pouvant être obtenu par le procédé conforme à l'invention ou d'une autre façon en un autre composé de formule I ou en l'un de ses tautomères, en ce que l'on sépare le mélange d'isomères pouvant être obtenu par le procédé conforme à l'invention en ses composants et/ou en ce que l'on transforme le composé libre de formule I ou l'un de ses tautomères pouvant être obtenu par le procédé conforme à l'invention en un sel ou le sel pouvant être obtenu par le procédé conforme à l'invention en un composé libre de formule I ou en l'un de ses tautomères ou en un autre sel.

**25.** Composé de formule

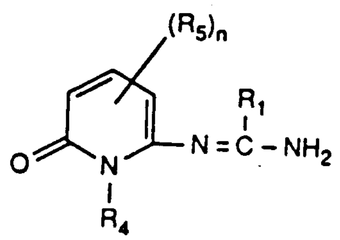

(III')

l'un de ses tautomères et/ou l'un de ses sels, où $R_1$, $R_4$, $R_5$ et n ont les significations données dans l'une des revendications 1 à 11.

**Revendications pour les Etats contractants suivants : AT, ES, GR**

**1.** Procédé pour la préparation des composés de formule

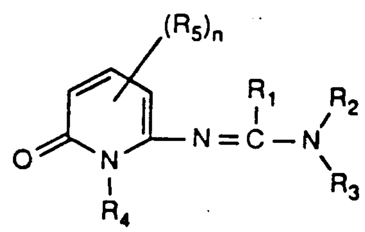

(I)

de leurs tautomères et/ou de leurs sels, où $R_1$ représente l'hydrogène ou un alkyle en $C_1$-$C_7$, l'un des restes $R_2$ et $R_3$ représente l'hydrogène, un alkyle en $C_1$-$C_7$, un phénylalkyle en $C_1$-$C_7$ ou un naphtylalkyle en $C_1$-$C_7$, un alcényle en $C_3$-$C_6$ ou un alcynyle en $C_3$-$C_6$ et l'autre reste un alkyle en $C_1$-$C_7$, un phénylalkyle en $C_1$-$C_7$ ou un naphtylalkyle en $C_1$-$C_7$, un alcényle en $C_3$-$C_6$ ou un alcynyle en $C_3$-$C_6$, $R_4$ l'hydrogène, un alkyle en $C_1$-$C_7$ ou un phénylalkyle en $C_1$-$C_7$ ou un naphtylalkyle en $C_1$-$C_7$, $R_5$ un alkyle en $C_1$-$C_7$ ou le trifluorométhyle et l'indice n est égal à 0, 1 ou 2, caractérisé

a) en ce que l'on fait réagir un composé de formule

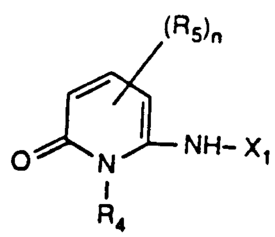

(IIa)

ou l'un de ses tautomères avec un composé de formule

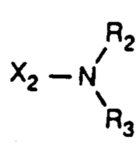

(IIb)

où l'un des restes $X_1$ et $X_2$ représente un groupe de formule -CO-$R_1$ et l'autre reste représente l'hydrogène ou un groupe de formule -CO-$Z_1$, $Z_1$ représentant un reste clivable ou avec l'un de ses tautomères, avec l'un de ses sels et/ou avec l'un de ses acétals ou

b) en ce que l'on transforme dans un composé de formule

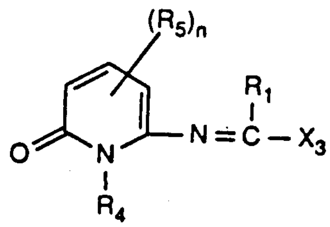

(III)

ou dans l'un de ses tautomères et/ou dans l'un de ses sels, où $X_3$ représente un reste transformable en

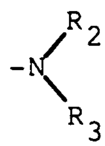

,

$X_3$ en

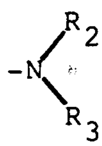

ou

c) en ce que l'on fait réagir un composé de formule

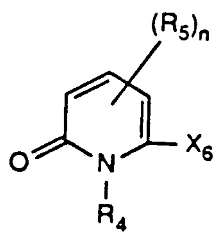

(Va)

ou l'un de ses tautomères ou l'un de ses sels avec un composé de formule

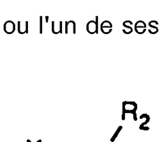

(Vb)

ou avec l'un de ses tautomères ou avec l'un de ses sels, $X_6$ représentant le groupe $-N=CR_1-NH-X_6$ et $X_7$ représentant l'hydrogène ou $X_6$ représentant $-NH_2$ et $X_7$ représentant le groupe $-CR_1=N-X_6$ et $X_6$ représentant un groupe partant et, si désiré, en ce que l'on transforme un composé de formule I ou l'un de ses tautomères pouvant être obtenu par le procédé conforme à l'invention ou d'une autre façon en un autre composé de formule I ou en l'un de ses tautomères, en ce que l'on sépare le mélange d'isomères pouvant être obtenu par le procédé conforme à l'invention en ses composants et/ou en ce que l'on transforme le composé libre de formule I ou l'un de ses tautomères pouvant être obtenu par le procédé conforme à l'invention en un sel ou le sel pouvant être obtenu par le

procédé conforme à l'invention en un composé libre de formule I ou en l'un de ses tautomères ou en un autre sel.

**2.** Procédé pour la préparation des composés de formule I

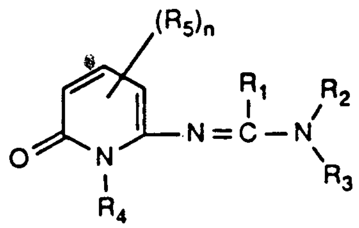

(I)

ou de leurs tautomères et/ou de leurs sels, où $R_1$ représente l'hydrogène ou un alkyle en $C_1$-$C_7$, l'un des restes $R_2$ et $R_3$ représente l'hydrogène, un alkyle en $C_1$-$C_7$, un phénylalkyle en $C_1$-$C_7$ ou un naphtylalkyle en $C_1$-$C_7$, un alcényle en $C_3$-$C_6$ ou un alcynyle en $C_3$-$C_6$ et l'autre reste un alkyle en $C_1$-$C_7$, un phénylalkyle en $C_1$-$C_7$ ou un naphtylalkyle en $C_1$-$C_7$, un alcényle en $C_3$-$C_6$ ou un alcynyle en $C_3$-$C_6$, $R_4$ l'hydrogène, un alkyle en $C_1$-$C_7$ ou un phénylalkyle en $C_1$-$C_7$ ou un naphtylalkyle en $C_1$-$C_7$, $R_5$ un alkyle en $C_1$-$C_7$ ou le trifluorométhyle et l'indice n est égal à 0, 1 ou 2, caractérisé en ce que, dans un composé de formule

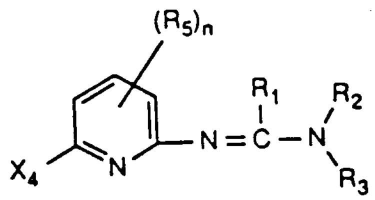

(IV)

ou dans l'un de ses sels, où $X_4$ représente un hydroxy protégé, l'on clive le groupe protecteur de l'hydroxy et, si désiré, en ce que l'on transforme un composé de formule I ou l'un de ses tautomères pouvant être obtenu par le procédé conforme à l'invention ou d'une autre façon en un autre composé de formule I ou en l'un de ses tautomères, en ce que l'on sépare le mélange d'isomères pouvant être obtenu par le procédé conforme à l'invention en ses composants et/ou en ce que l'on transforme le composé libre de formule I ou l'un de ses tautomères pouvant être obtenu par le procédé conforme à l'invention en un sel ou le sel pouvant être obtenu par le procédé conforme à l'invention en un composé libre de formule I ou en l'un de ses tautomères ou en un autre sel.

**3.** Procédé selon la revendication 1 ou 2 pour la préparation des composés de formule I ou de leurs tautomères et/ou de leurs sels, où $R_1$ représente l'hydrogène ou un alkyle en $C_1$-$C_7$, l'un des restes $R_2$ et $R_3$ représente l'hydrogène, un alkyle en $C_1$-$C_7$ ou un phényl- ou naphtylalkyle en $C_1$-$C_7$ et l'autre reste représente un alkyle en $C_1$-$C_7$ ou un phényl- ou naphtylalkyle en $C_1$-$C_7$, $R_4$ représente l'hydrogène ou un alkyle en $C_1$-$C_7$, $R_5$ représente un alkyle en $C_1$-$C_7$ et l'indice n est 0 ou 1.

**4.** Procédé selon la revendication 1 ou 2 pour la préparation des composés de formule I ou de leurs tautomères et/ou de leurs sels, où $R_1$ représente l'hydrogène ou un alkyle en $C_1$-$C_7$, l'un des restes $R_2$ et $R_3$ représente l'hydrogène, un alkyle en $C_1$-$C_7$ ou un phényl- ou naphtylalkyle en $C_1$-$C_7$ et l'autre reste représente un alkyle en $C_1$-$C_7$ ou un phényl- ou naphtylalkyle en $C_1$-$C_7$, $R_4$ représente l'hydrogène et l'indice n est 0.

**5.** Procédé selon l'une des revendications 1 à 4 pour la préparation des composés de formule I ou de leurs tautomères et/ou de leurs sels, où l'un des restes $R_2$ et $R_3$ représente l'hydrogène, un alkyle en $C_1$-$C_7$ ou un phényl- ou naphtylalkyle en $C_1$-$C_7$ et l'autre reste représente un alkyle en $C_1$-$C_7$ ou un phényl- ou naptylalkyle en $C_1$-$C_7$.

**6.** Procédé selon la revendication 4 pour la préparation des composés de formule I ou de leurs

tautomères et/ou de leurs sels, où $R_1$ représente l'hydrogène ou un alkyle en $C_1$-$C_4$, $R_2$ et $R_3$ représentent indépendamment l'un de l'autre, un alkyle en $C_1$-$C_4$ ou $R_2$ représente un alkyle en $C_1$-$C_4$ et $R_3$ représente un phénylalkyle en $C_1$-$C_4$, $R_4$ est l'hydrogène et l'indice n est 0.

**7.** Procédé selon la revendication 1 pour la préparation des composés de formule I ou de leurs tautomères et/ou de leurs sels, où $R_1$ représente l'hydrogène ou un alkyle en $C_1$-$C_4$, $R_2$ et $R_3$ représentent, indépendamment l'un de l'autre, un alkyle en $C_1$-$C_4$ ou $R_2$ représente un alkyle en $C_1$-$C_4$ et $R_3$ représente un phénylalkyle en $C_1$-$C_4$, $R_4$ est un alkyle en $C_1$-$C_4$ et l'indice n est égal à 0.

**8.** Procédé selon la revendication 4 pour la préparation des composés de formule I ou de leurs tautomères et/ou de leurs sels, où $R_1$ et $R_4$ représentent l'hydrogène, $R_2$ et $R_3$ représentent, indépendamment l'un de l'autre, un alkyle en $C_1$-$C_4$ et l'indice n est 0.

**9.** Procédé selon la revendication 1 pour la préparation des composés de formule I ou de leurs tautomères et/ou de leurs sels, où $R_1$ représente l'hydrogène, $R_4$ un alkyle en $C_1$-$C_4$, $R_2$ et $R_3$ représentent, indépendamment l'un de l'autre, un alkyle en $C_1$-$C_4$ et l'indice n est 0.

**10.** Procédé selon la revendication 3 pour la préparation des composés de formule I ou de leurs tautomères et/ou de leurs sels, où $R_1$ est le méthyle, $R_4$ représente l'hydrogène, $R_2$ et $R_3$ représentent, indépendamment l'un de l'autre, un alkyle en $C_1$-$C_4$ et l'indice n est 0.

**11.** Procédé selon la revendication 6 pour la préparation des composés de formule I ou de leurs tautomères et/ou de leurs sels, où $R_1$ représente l'hydrogène ou le méthyle, $R_2$ le méthyle, $R_3$ le 2-phényléthyle, $R_4$ l'hydrogène et l'indice n est 0.

**12.** Procédé selon la revendication 7 pour la préparation des composés de formule I ou de leurs tautomères et/ou de leurs sels, où $R_1$ représente l'hydrogène ou le méthyle, $R_2$ le méthyle, $R_3$ le 2-phényléthyle, $R_4$ est le méthyle et l'indice n est 0.

**13.** Procédé selon la revendication 1 ou 2 pour la préparation de la N-(2-hydroxy-6-pyridyl)-N',N'-di-n-propylformamidine ou de la N-(2-pyridon-6-yl)-N',N'-di-n-propyl-formamidine ou de l'un de leurs sels.

**14.** Procédé selon la revendication 1 ou 2 pour la préparation de la N-(2-hydroxy-6-pyridyl)-N',N'-di-n-propyl-acétamidine ou de la N-(2-pyridon-6-yl)-N',N'-di-n-propylacétamidine ou de l'un de leurs sels.

**15.** Procédé selon la revendication 1 ou 2 pour la préparation de la N-(2-hydroxy-6-pyridyl)-N'-méthyl-N'-(2-phényléthyl)-acétamidine ou de la N-(2-pyridon-6-yl)-N'-méthyl-N'-(2-phényléthyl)-acétamidine ou de l'un de leurs sels.

**16.** Procédé selon la revendication 1 pour la préparation de la N-(1-méthyl-2-pyridon-6-yl)-N',N'-di-n-propylformamidine ou de l'un de ses sels.

**17.** Procédé selon la revendication 1 pour la préparation de la N-(1-méthyl-2-pyridon-6-yl)-N',N'-dipropyl-acétamidine ou de l'un de ses sels.

**18.** Procédé selon la revendication 1 pour la préparation de la N-(1-benzyl-2-pyridon-6-yl)-N',N'-dipropyl-formamidine ou de l'un de ses sels.

**19.** Procédé pour la préparation des composés de formule

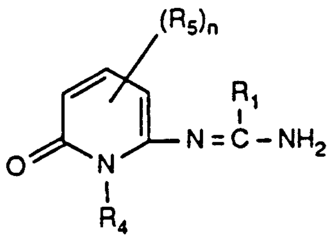

(III')

ou de leurs tautomères et/ou de leurs sels, où $R_1$, $R_4$, $R_5$ et n ont les significations données dans l'une des revendications 1 à 12, caractérisé en ce que l'on fait réagir un composé de formule

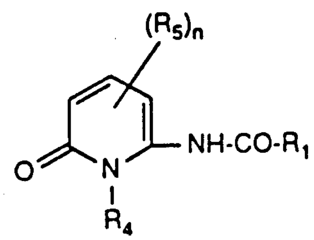

(IIIa)

ou l'un de ses tautomères et/ou l'un de ses sels avec l'ammoniac.

**20.** Procédé pour l'obtention d'une préparation pharmaceutique, caractérisé en ce que l'on met sous forme de préparation pharmaceutique un composé pouvant être obtenu selon l'une des revendications 1 à 18 ou l'un de ses tautomères, sous forme libre ou sous forme d'un sel pharmaceutique utilisable, éventuellement en mélangeant des adjuvants pharmaceutiques usuels.

**21.** Procédé pour l'obtention d'une préparation pharmaceutique, caractérisé
a) en ce que l'on fait réagir un composé de formule

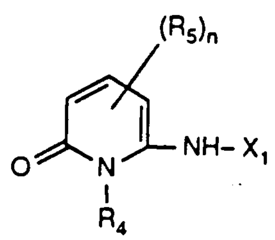

(IIa)

ou l'un de ses tautomères avec un composé de formule

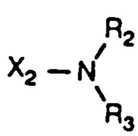

(IIb)

où l'un des restes $X_1$ et $X_2$ représente un groupe de formule $-CO-R_1$ et l'autre reste représente l'hydrogène ou un groupe de formule $-CO-Z_1$, $Z_1$ représentant un reste clivable ou avec l'un de ses tautomères, avec l'un de ses sels et/ou avec l'un de ses acétals ou
b) en ce que l'on transforme dans un composé de formule

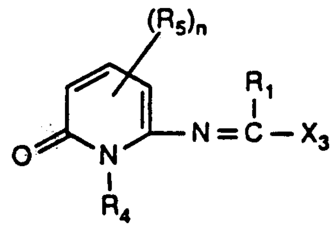

(III)

ou dans l'un de ses tautomères et/ou dans l'un de ses sels, où $X_3$ représente un reste transformable en

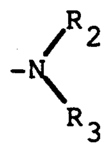

,

$X_3$ en

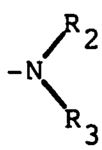

ou

c) en ce que l'on fait réagir un composé de formule

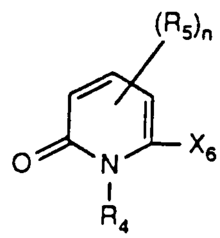

(Va)

ou l'un de ses tautomères ou l'un de ses sels avec un composé de formule

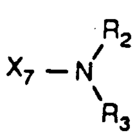

(Vb)

ou avec l'un de ses tautomères ou avec l'un de ses sels, $X_6$ représentant le groupe $-N=CR_1-NH-X_6$ et $X_7$ représentant l'hydrogène ou $X_6$ représentant $-NH_2$ et $X_7$ représentant le groupe $-CR_1=N-X_6$ et $X_6$ représentant un groupe partant et, si désiré, en ce que l'on transforme un composé de formule I ou l'un de ses tautomères pouvant être obtenu par le procédé conforme à l'invention ou d'une autre façon en un autre composé de formule I ou en l'un de ses tautomères, en ce que l'on sépare le mélange d'isomères pouvant être obtenu par le procédé conforme à l'invention en ses composants et/ou en ce que l'on transforme le composé libre de formule I ou l'un de ses tautomères pouvant être obtenu par le procédé conforme à l'invention en un sel ou le sel pouvant être obtenu par le procédé conforme à l'invention en un composé libre de formule I ou en l'un de ses tautomères ou

en un autre sel, et en ce que l'on met sous forme de préparation pharmaceutique le composé obtenu de cette façon de formule

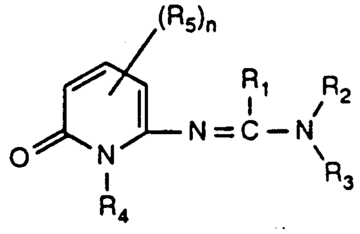

(I)

où $R_1$ représente l'hydrogène ou un alkyle en $C_1$-$C_7$, l'un des restes $R_2$ et $R_3$ représente l'hydrogène, un alkyle en $C_1$-$C_7$, un phénylalkyle en $C_1$-$C_7$ ou un naphtylalkyle en $C_1$-$C_7$, un alcényle en $C_3$-$C_6$ ou un alcynyle en $C_3$-$C_6$, et l'autre reste représente un alkyle en $C_1$-$C_7$, un phénylalkyle en $C_1$-$C_7$ ou un naphtylalkyle en $C_1$-$C_7$, un alcényle en $C_3$-$C_6$ ou un alcynyle en $C_3$-$C_6$, $R_4$ représente l'hydrogène, un alkyle en $C_1$-$C_4$ ou un phénylalkyle en $C_1$-$C_7$ ou un naphtylalkyle en $C_1$-$C_7$, $R_5$ représente un alkyle en $C_1$-$C_7$ ou le trifluorométhyle et l'indice n représente 0, 1 ou 2 ou l'un de ses tautomères obtenu de cette façon, sous forme libre ou sous forme d'un sel pharmaceutiquement utilisable, éventuellement en mélangeant avec des adjuvants pharmaceutiques usuels.

**22.** Procédé pour l'obtention d'une préparation pharmaceutique, caractérisé en ce que, dans un composé de formule

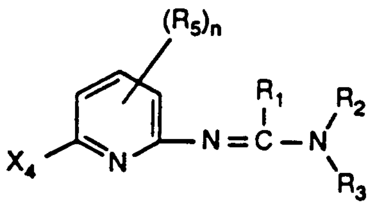

(IV)

ou dans l'un de ses sels où $X_4$ représente un hydroxy protégé, l'on clive le groupe protecteur de l'hydroxy, et, si désiré, en ce que l'on transforme un composé de formule I ou l'un de ses tautomères pouvant être obtenu par le procédé conforme à l'invention ou d'une autre façon en un autre composé de formule I ou en l'un de ses tautomères, en ce que l'on sépare le mélange d'isomères pouvant être obtenu par le procédé conforme à l'invention en ses composants et/ou en ce que l'on transforme le composé libre de formule I ou l'un de ses tautomères pouvant être obtenu par le procédé conforme à l'invention en un sel ou le sel pouvant être obtenu par le procédé conforme à l'invention en un composé libre de formule I ou en l'un de ses tautomères ou en un autre sel et en ce que l'on met sous forme de préparation pharmaceutique le composé obtenu de cette façon de formule

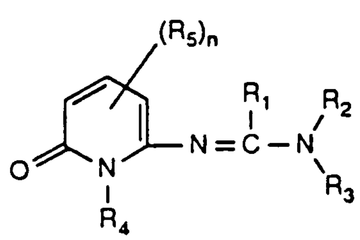

(I)

où $R_1$ représente l'hydrogène ou un alkyle en $C_1$-$C_7$, l'un des restes $R_2$ et $R_3$ représente l'hydrogène, un alkyle en $C_1$-$C_7$, un phénylalkyle en $C_1$-$C_7$ ou un naphtylalkyle en $C_1$-$C_7$, un alcényle en $C_3$-$C_6$ ou un alcynyle en $C_3$-$C_6$, et l'autre reste représente un alkyle en $C_1$-$C_7$, un phénylalkyle en $C_1$-$C_7$ ou un naphtylalkyle en $C_1$-$C_7$, un alcényle en $C_3$-$C_6$ ou un alcynyle en $C_3$-$C_6$, $R_4$ représente l'hydrogène, un

alkyle en $C_1$-$C_7$ ou un phénylalkyle en $C_1$-$C_7$ ou un naphtylalkyle en $C_1$-$C_7$, $R_5$ représente un alkyle en $C_1$-$C_7$ ou le trifluorométhyle et l'indice n représente 0, 1 ou 2 ou l'un de ses tautomères obtenu de cette façon, sous forme libre ou sous forme d'un sel pharmaceutique utilisable, éventuellement en mélangeant avec des adjuvants pharmaceutiques usuels.

**23.** Procédé selon l'une des revendications 20 à 22 pour l'obtention d'une préparation pharmaceutique nootrope, antidépresseur et antiparkinsonien, caractérisé en ce que l'on choisit une substance active agissant comme nootrope, antidépresseur et antiparkinsonien.

**24.** Utilisation d'un composé ou de l'un de ses tautomères pouvant être obtenu selon l'une des revendications 1 à 18, sous forme libre ou sous forme d'un sel pharmaceutiquement utilisable, pour l'obtention d'une préparation pharmaceutique.

**25.** Utilisation d'un composé ou de l'un de ses tautomères selon la revendication 24 pour l'obtention d'une préparation pharmaceutique agissant comme nootrope, antidépresseur et antiparkinsonien.